(19) Europäisches Patentamt
European Patent Office
Office européen des brevets

(11) Veröffentlichungsnummer: **0 088 940**
**B1**

# (12) EUROPÄISCHE PATENTSCHRIFT

(45) Veröffentlichungstag der Patentschrift: 21.05.86

(21) Anmeldenummer: 83102001.1

(22) Anmeldetag: 02.03.83

(51) Int. Cl.4: C 07 D 213/80, C 07 D 213/85, C 07 D 491/04, A 61 K 31/455 // (C07D491/04, 307:00, 221:00)

(54) **Pyridincarbonsäureester, Verfahren zu ihrer Herstellung sowie ihre Verwendung als Arzneimittel.**

(30) Priorität: 13.03.82 DE 3209274

(43) Veröffentlichungstag der Anmeldung: 21.09.83 Patentblatt 83/38

(45) Bekanntmachung des Hinweises auf die Patenterteilung: 21.05.86 Patentblatt 86/21

(84) Benannte Vertragsstaaten: AT BE CH DE FR GB IT LI LU NL SE

(56) Entgegenhaltungen:
DE - A - 2 629 892
ARZNEIMITTEL-FORSCHUNG, Band 24, Nr. 4, April 1974, Seiten 455-466, Editio Cantor KG., Aulendorf, DE., H. ROSENKRANZ et al.: "Die Bindung von 4-(2'-Nitrophenyl)-2,6-dimethyl-1,4-dihydropyridin-3,5-dicarbonsäuredimethylester (Nifedipine) sowie von anderen koronarwirksamen Stoffen an die Eiweisskörper des Serums"
ARZNEIMITTEL-FORSCHUNG, Band 22, Nr. 1, April 1972, Seiten 60-62, Editio Cantor KG., Aulendorf, DE. K. SCHLOSSMANN: "Fluorimetrische Bestimmung des 4-(2'-Nitrophenyl)-2,6-dimethyl-1,4-dihydropyridine-3,5-dicarbonsäuredimethylester und seines Hauptmetaboliten"
CHEMICAL ABSTRACTS, Band 68, Nr. 21, 20. Mai 1968, Seite 9222, Nr. 95651d, Columbus, Ohio, USA, TETSUJI KAMETANI et al.: "Syntheses of heterocyclic compounds. CXCVIII. Streptonigrin and related compounds. 7. Synthesis of the streptonigrin nucleus by the formation of a pyridine ring"
CHEMICAL ABSTRACTS, Band 79, Nr. 23, 10. Dezember 1973, Seite 6, Nr. 13381f, Columbus, Ohio, USA, S.S. WALKENSTEIN et al.: "Metabolism of the antihypertensive agent

(73) Patentinhaber: BAYER AG, Konzernverwaltung RP Patentabteilung, D-5090 Leverkusen 1 Bayerwerk (DE)

(72) Erfinder: Wehinger, Egbert, Dr., Gellertweg 33, D-5600 Wuppertal 1 (DE)
Erfinder: Meyer, Horst, Dr., Henselweg 11, D-5600 Wuppertal 1 (DE)
Erfinder: Benz, Ulrich, Gustav-Poensgenstrasse 29, D-4000 Düsseldorf 1 (DE)

(56) Entgegenhaltungen: (Fortsetzung)
1,4-dihydro-2,6-dimethyl-4-(2-trifluoromethylphenyl)-3,5-pyridinedicarboxylic acid diethyl ester"
CHEMICAL ABSTRACTS, Band 84, Nr. 25, 21. Juni 1976, Seite 17, Nr. 173595k, Columbus, Ohio, USA, K. SCHLOSSMANN et al.: "Investigations on the metabolism and protein binding of nifedipine"
JOURNAL OF THE CHEMICAL SOCIETY, Nr. 191, 1946, Seiten 884-888, Columbus, Ohio, USA, V.A. PETROW: "New syntheses of heterocyclic compounds. Part VII. 9-Amino-6: 8-dimethyl-7: 10-diazaphenanthrenes"
JOURNAL OF MEDICINAL CHEMISTRY, Band 16, Nr. 1, 1973, Seiten 34-37, Columbus, Ohio, USA, S.E. PARKER et al.: "Biotransformation of 1,4-dihydro-2,6-dimethyl-4-(2-trifluoromethylphenyl)-3,5-pyridinedicarboxylic acid diethyl ester"
JOURNAL OF HETEROCYCLIC CHEMISTRY, Band 12, 1975, Seiten 363-365, Columbus, Ohio, USA, B. LOEV et al.: "Hantzsch-Type" dihydropyridines. IV (1). Carboxylic acids"
ARZNEIMITTEL-FORSCHUNG, Band 32, Nr. 4, April 1982, Seiten 338-346, Editio Cantor KG., Aulendorf, DE., R. TOWART et al.: "The effects of nimodipine, its optical isomers and metabolites on isolated vascular smooth muscle"

EP 0 088 940 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue Pyridincarbonsäureester, mehrere Verfahren zu ihrer Herstellung sowie ihre Verwendung in Arzneimitteln, insbesondere in Mitteln gegen Erkrankungen, die auf einer Ischämie und/oder einer Hypoxie beruhen.

Es ist bereits bekannt geworden, daß man 2,6-Dimethyl-4-(2-nitrophenyl)-pyridin-3,5-dicarbonsäurediethylester durch Chromsäureoxidation des entsprechenden 1,4-Dihy-dro-2,6-dimethyl-4-(2-nitrophenyl)-pyridin-3,5-dicarbon-säurediethylesters erhält (s. V.A. Petrow, J.Chem.Soc. 884 (1946)).

Weiterhin ist bekannt geworden, daß bei der Biotransformation von vasodilatierend wirksamen 4-Aryl-1,4-dihydropyridinderivaten Pyridine entstehen, die wesentlich schwächer gefäßwirksam sind als die entsprechenden Dihydropyridinverbindungen (s. S. Higuchi et al. 95. General Congress of the Japanese Pharmaceutical Society, April 1975; S.E. Parker und J. Weinstock, J.Med.Chem. 16, 34 (1973); H. Medenwald, K. Schloßmann und C. Wünsche, Arzneim.-Forsch. 22, 53 (1972)).

Weiterhin ist bekannt geworden, daß die Verbindung 1,4-Dihydro-2,6-dimethyl-4-(3-nitrophenyl)-pyridin-3,5-di-carbonsäure-isopropyl-(2-methoxyethyl)-ester experimentell induzierte Lern- und Gedächtnisstörungen deutlich verbessert (vgl. DOS 2 815 578).

Weiterhin sind 4-(3-Nitrophenyl)-pyridin-3,5-dicarbonsäureester als Zwischenprodukte bei der Synthese von antibakteriell wirksamen Pyridylchinolonen verwendet worden (P.M. Carbateas und G.L. Williams, J. Heterocyclic Chem. 11, 819 (1974)).

Die vorliegende Erfindung betrifft neue Pyridincarbonsäureester der allgemeinen Formel (I)

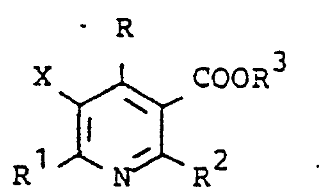

(I)

in welcher

R für einen Phenyl- oder Pyridylrest steht, wobei der Phenylrest gegebenenfalls 1- oder 2-fach substituiert ist durch Nitro, Cyano, Trifluormethyl, Fluor, Chlor, Brom, Alkyl, Alkoxy, Alkylmercapto mit jeweils 1 bis 4 Kohlenstoffatomen in den Alkyl- und Alkoxyresten, oder für einen Benzoxadiazolylrest steht,

$R^1$ für Wasserstoff oder einen Alkylrest mit 1 bis 4 Kohlenstoffatomen, die Phenylgruppe, die Benzylgruppe, die Acetoxymethylgruppe, die 2-Acetoxyethylgruppe oder eine Hydroxyalkylgruppe mit 1 bis 4 Kohlenstoffatomen steht, oder

$R^1$ zusammen mit X einen 5- bis 7-gliedrigen Ring bilden, der als Ringglied eine Carbonylgruppe und gegebenenfalls ein Sauerstoff- oder Stickstoffatom enthält, wobei der Stickstoff gegebenenfalls noch einen Wasserstoff oder einen niederen Alkylrest mit 1 bis 4 C-Atomen trägt,

$R^2$ für Wasserstoff oder für einen geradkettigen oder verzweigten Alkylrest mit 1 bis 4 Kohlenstoffatomen, Phenyl, Benzyl, Acetoxymethyl, 2-Acetoxyethyl oder Hydroxyalkyl mit 1 bis 4 Kohlenstoffatomen steht,

X für eine Nitrilgruppe steht

oder

für den Rest $COR^4$ steht, wobei $R^4$ einen geradkettigen oder verzweigten Alkylrest mit 1 bis 4 Kohlenstoffatomen, Phenyl oder Benzyl darstellt

oder

für die Gruppe $COOR^5$ steht, wobei $R^5$ einen geradkettigen, verzweigten oder cyclischen gesättigten oder ungesättigten Kohlenwasserstoffrest mit bis zu 10 Kohlenstoffatomen darstellt, der gegebenenfalls durch ein Sauerstoffatom in der Kette unterbrochen ist und/ oder der gegebenenfalls substituiert ist durch Fluor, Chlor, Brom, Cyano, Hydroxy, Acetoxy, α-, β- oder γ-Pyridyl, Phenyl, Phenoxy, wobei die Arylgruppen ihrerseits durch Fluor, Chlor, Nitro, Trifluormethyl, Alkyl, Alkoxy mit jeweils 1 bis 4 Kohlenstoffatomen oder Amino substituiert ist, wobei die Aminogruppe ihrerseits gegebenenfalls durch zwei gleiche oder verschiedene Substituenten aus der Gruppe Alkyl mit 1 bis 4 Kohlenstoffatomen, Phenyl oder Benzyl substituiert ist oder wobei $R^5$ auch Wasserstoff sein kann, wenn $R^1$ eine Hydroxyalkyl- oder Acyloxyalkylgruppe darstellt,

oder

für die Gruppe $SO_2R^6$ steht, wobei $R^6$ einen Alkylrest mit 1 bis 4 Kohlenstoffatomen oder Phenyl darstellt, welches gegebenenfalls durch Fluor, Chlor, Trifluormethyl, Alkyl oder Alkoxy mit jeweils 1 bis 2 Kohlenstoffatomen substituiert ist,

$R^3$ immer verschieden von $R^5$ ist und für einen geradkettigen Kohlenwasserstoffrest mit bis zu 6 Kohlenstoffatomen steht, der durch ein Sauerstoffatom in der Kette unterbrochen ist und/oder der substituiert ist durch Fluor, Chlor, Brom, Cyano, Hydroxy, Acetoxy, Dialkylamino mit bis zu 4 Kohlenstoffatomen je Alkylgruppe, α -, ßoder γ -Pyridyl, Phenyl oder Phenoxy, wobei die Arylgruppen ihrerseits durch Trifluormethyl, Fluor, Chlor, Nitro, Alkyl oder Alkoxy mit jeweils 1 bis 4 Kohlenstoffatomen substituiert sein können, sowie ihre pharmazeutisch unbedenklichen Salze.

Überraschenderweise zeigen die erfindungsgemäßen Verbindungen eine starke Hypoxieschutzwirkung, die sowohl bei einer Hypoxie-induzierten Amnesie als auch bei der Hypoxietoleranz zum Ausdruck kommt. Aus dem Stand der Technik konnten diese speziellen pharmakologischen Wirkungen nicht erwartet werden. Die erfindungsgemäßen neuen Verbindungen sind aufgrund dieser Eigenschaften als Bereicherung der Pharmazie anzusehen.

Weiterhin wurde gefunden, daß man die erfindungsgemäßen Verbindungen der Formel (I) erhält, wenn man

A) 1,4-Dihydropyridinderivate der allgemeinen Formel (II)

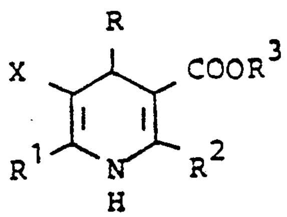

(II)

in welcher

R, $R^1$, $R^2$, $R^3$ und X die oben angegebene Bedeutung haben,

mit oxidierenden (dehydrierenden) Agentien gegebenenfalls in Gegenwart inerter Lösungsmittel bei Temperaturen zwischen 0 und 200°C zur Reaktion bringt,

oder

B) Pyridincarbonsäuren der allgemeinen Formel (III)

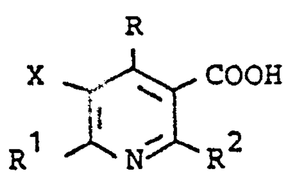

(III)

in welcher

R, $R^1$, $R^2$ und X die oben angegebene Bedeutung haben, gegebenenfalls nach Aktivierung der Carboxylgruppe entsprechend literaturbekannten Methoden zu Carbonsäureestern der allgemeinen Formel (I) verestert,

oder

C) für den Fall, daß $R^1$ und/oder $R^2$ in der allgemeinen Formel (I) eine Hydroxyalkylgruppe darstellen, die entsprechenden Mono- bzw. Bisacetoxyalkylpyridine den Bedingungen einer milden und selektiven Hydrolyse unterwirft,

oder

D) für den Fall, daß X in der Formel (I) Carboxyl bedeutet und $R^1$ Hydroxyalkyl bedeutet, Verbindungen der Formel (IV)

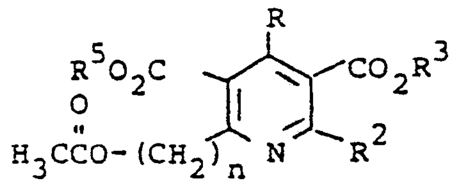

(IV)

in welcher

R, $R^2$, $R^3$, $R^5$ und n die oben angegebene Bedeutung haben,

in Gegenwart von Alkali zu Verbindungen der Formel (V) verseift,

oder

E) für den Fall, daß $R^1$ mit X in der allgemeinen Formel (I) einen Lactonring bildet, Hydroxyalkylpyridincarbonsäuren der allgemeinen Formel (V)

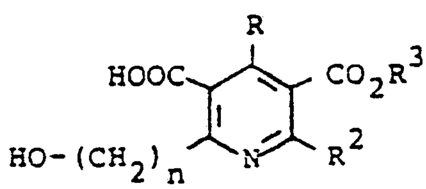

(V)

in welcher

R, $R^2$ und $R^3$ die oben angegebene Bedeutung haben

und

n eine Zahl von 1 bis 4 darstellt,

unter dem Einfluß von Protonen nach üblichen Methoden zu Verbindungen der Formel (VI)

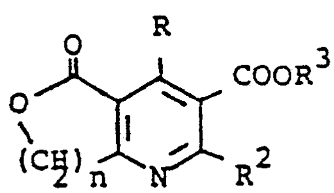

(VI)

in welcher

R, $R^2$, $R^3$ und n die oben angegebene Bedeutung haben,

cyclisiert.

Die erfindungsgemäßen Verbindungen der

allgemeinen Formel (I) können je nach Art der Substituenten auf verschiedene Weisen hergestellt werden.

Besonders vorteilhaft und am breitesten anwendbar erwies sich gemäß Verfahren A die Umsetzung von 1,4-Dihydropyridinderivaten der allgemeinen Formel (II)

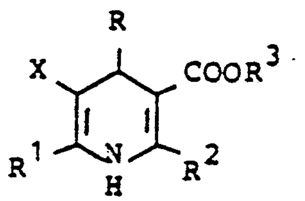

(II)

mit oxidierenden (dehydrierenden) Agentien.

In der Formel (II) haben die Reste R, $R^1$, $R^2$, $R^3$ und X die oben angegebene Bedeutung.

Die als Ausgangsmaterial eingesetzten 1,4-Dihydropyridinderivate der Formel (II) sind literaturbekannt oder können nach literaturbekannten Methoden hergestellt werden (s. z.B. DOS 2 117 571, DOS 2 508 181, DOS 2 847 236).

Als Beispiele seien genannt:

1,4-Dihydro-2,6-dimethyl-4-(2-nitrophenyl)-pyridin 3,5-dicarbonsäure-(2-hydroxyethyl)-methyl-ester,

1,4-Dihydro-2,6-dimethyl-4-(2-nitrophenyl)-pyridin 3,5-dicarbonsäure-(2-chlorethyl)-isopropyl-ester,

1,4-Dihydro-2,6-dimethyl-4-(2-nitrophenyl)-pyridin 3,5-dicarbonsäure-(2-cyanoethyl)-methyl-ester,

1,4-Dihydro-2,6-dimethyl-4-(2-nitrophenyl)-pyridin 3,5-dicarbonsäure-(2-acetoxyethyl)-isopropyl-ester,

1,4-Dihydro-2,6-dimethyl-4-(3-nitrophenyl)-pyridin-3,5-dicarbonsäure-(2-acetoxyethyl)-methyl-ester,

1,4-Dihydro-2,6-dimethyl-4-(3-nitrophenyl)-pyridin-3,5-dicarbonsäure-(2-phenoxyethyl)-ethyl-ester,

1,4-Dihydro-2,6-dimethyl-4-(3-nitrophenyl)-pyridin-3,5-dicarbonsäure-(2-hydroxyethyl)-(2-N-benzyl-N-methylaminoethyl)-ester,

1,4-Dihydro-2,6-dimethyl-4-(3-nitrophenyl)-pyridin-3,5-dicarbonsäure-(3-hydroxypropyl)-isopropyl-ester,

3-Acetyl-1,4-dihydro-2,6-dimethyl-4-(3-nitrophenyl)-pyridin-5-carbonsäure-(2-hydroxyethyl)-ester,

3-Acetyl-1,4-dihydro-2,6-dimethyl-4-(2-trifluormethyl-phenyl)-pyridin-5-carbonsäure-(2-hydroxyethyl)-ester,

3-Acetyl-1,4-dihydro-2,6-dimethyl-4-(2-trifluormethyl-phenyl)-pyridin-5-carbonsäure-(2-cyanoethyl)-ester,

3-Acetyl-1,4-dihydro-2,6-dimethyl-4-(2-trifluormethyl-phenyl)-pyridin-5-carbonsäure-(2-acetoxyethyl)-ester,

3-Cyano-1,4-dihydro-2,6-dimethyl-4-(3-nitrophenyl)-pyridin -5-carbonsäure-(2-hydroxyethyl)-ester,

3-Cyano-1,4-dihydro-2,6-dimethyl-4-(trifluormethylphenyl) -pyridin-5-carbonsäure-(2-hydroxyethyl)-ester,

3-Cyano-1,4-dihydro-2,6-dimethyl-4-(2-methoxyphenyl)-pyridin-5-carbonsäure-(2-hydroxyethyl)-ester,

3-Cyano-1,4-dihydro-2,6-dimethyl-4-(2-chlorphenyl)-pyridin-5-carbonsäure-(2-acetoxyethyl)-ester,

1,4-Dihydro-2,6-dimethyl-4-(pyridyl-2)-pyridin-3,5-dicarbonsäure-(2-hydroxyethyl)-isopropyl-ester,

1,4-Dihydro-2,6-dimethyl-4-(pyridyl-3)-pyridin-3,5-dicarbonsäure-(2-hydroxyethyl)-isopropyl-ester,

1,4-Dihydro-2,6-dimethyl-4-(2,1,3-benzoxadiazolyl-4)-; pyridin-3,5-dicarbonsäure-(2-hydroxyethyl)-methylester,

1,4-Dihydro-2,6-dimethyl-4-(2,1,3-benzoxadiazolyl-4)-pyridin-3,5-dicarbonsäure-(2-cyanoethyl)-isopropylester.

Als Oxidationsmittel (Dehydrierungsmittel) kommen in erster Linie Salpetersäure oder salpetrige Säure, Chrom-(VI)-oxid oder Natriumdichromat, Stickoxide, Chloranil, Tetracyanobenzochinon oder die anodische Oxidation in Gegenwart eines geeigneten Elektrolytsystems wie beispielsweise Acetonitril/Lithiumperchlorat in Frage.

Als Verdünnungsmittel seien in erster Linie Wasser und alle inerten organischen Lösungsmittel genannt. Hierzu gehören vorzugsweise Benzol, Toluol, Acetonitril, Eisessig und Hexamethylphosphorsäuretriamid.

Die Reaktionstemperaturen können in einem größeren Bereich variiert werden. Im allgemeinen arbeitet man zwischen 0 und 200°C, vorzugsweise so, daß die Reaktion gut gesteuert werden kann.

Je nach Art der Substituenten kann es vorteilhaft sein, als letzten Syntheseschritt nicht die Umwandlung des 1,4-Dihydropyridinderivates der allgemeinen Formel (II) in die erfindungsgemäßen Verbindungen der allgemeinen Formel (I) vorzunehmen.

So können auch gemäß Verfahrensvariante B Pyridincarbonsäuren der allgemeinen Formel (III)

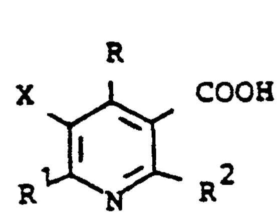

(III)

in welcher
R, $R^1$, $R^2$ und X die oben angegebene Bedeutung haben,

nach literaturbekannten Methoden in die erfindungsgemäßen Pyridincarbonsäureester der allgemeinen Formel (I) überführt werden. Es ist auch möglich, die erfindungsgemäßen Verbindungen der allgemeinen Formel (VII)

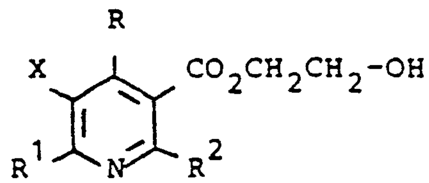

(VII)

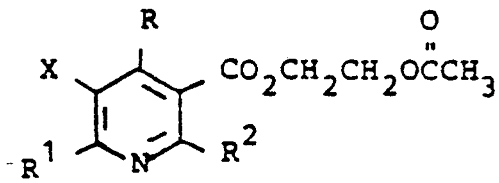

(VIII)

zu erfindungsgemäßen Verbindungen der allgemeinen Formel (VIII) zu acylieren, wobei R, $R^1$, $R^2$ und X die oben angegebene Bedeutung haben (s. Beispiel 4).

Die vorstehenden Herstellungsverfahren sind lediglich zur Verdeutlichung angegeben, und die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Je nach der Wahl der Substituenten können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z.B. E.L. Eliel, Stereochemistry of Carbon Compounds, McGraw Hill, 1962).

Außer den unten angeführten Herstellungsbeispielen seien folgende erfindungsgemäße Wirkstoffe genannt:

2,6-Dimethyl-4-(2-nitrophenyl)-pyridin-3,5-dicarbon-säure-(2-hydroxyethyl)-methyl-ester,

2,6-Dimethyl-4-(2-nitrophenyl)-pyridin-3,5-dicarbon-saure-(2-hydroxyethyl)-ethyl-ester,

2,6-Dimethyl-4-(2-nitrophenyl)-pyridin-3,5-dicarbon-säure-(2-hydroxyethyl)-isobutyl-ester,

2,6-Dimethyl-4-(2-nitrophenyl)-pyridin-3,5-dicarbon-säure-(2-hydroxyethyl)-cyclopentyl-ester,

2,6-Dimethyl-4-(3-nitrophenyl)-pyridin-3,5-dicarbon-säure-(3-hydroxypropyl)-isopropyl-ester,

2,6-Dimethyl-4-(3-nitrophenyl)-pyridin-3,5-dicarbon-säure-(3-acetoxypropyl)-isopropyl-ester,

2,6-Dimethyl-4-(3-nitrophenyl)-pyridin-3,5-dicarbon-säure-(3-chlorpropyl)-isopropyl-ester,

2,6-Dimethyl-4-(3-nitrophenyl)-pyridin-3,5-dicarbon-säure-(3-cyanopropyl)-isopropyl-ester,

2,6-Dimethyl-4-(3-nitrophenyl)-pyridin-3,5-dicarbon-säure-(4-hydroxybutyl)-isopropyl-ester,

2,6-Dimethyl-4-(3-nitrophenyl)-pyridin-3,5-dicarbon-säure-(2-hydroxyethyl)-benzyl-ester,

2,6-Dimethyl-4-(3-nitrophenyl)-pyridin-3,5-dicarbon-säure-(2-hydroxyethyl)-(2,2,2-trifluorethyl)-ester,

2,6-Dimethyl-4-(3-nitrophenyl)-3-acetyl-pyridin-5-carbonsäure-(2-hydroxyethyl)-ester,

2,6-Dimethyl-4-(3-nitrophenyl)-3-acetyl-pyridin-5-carbonsäure-(2-acetoxyethyl)-ester,

2,6-Dimethyl-4-(3-nitrophenyl)-3-acetyl-pyridin-5-carbonsäure-(2-chlorethyl)-ester,

2,6-Dimethyl-4-(2-chlorphenyl)-pyridin-3,5-dicarbon-säure-(2-hydroxyethyl)-isopropyl-ester,

2,6-Dimethyl-4-(2,3-dichlorphenyl)-pyridin-3,5-di-carbonsäure-(2-hydroxyethyl)-isopropyl-ester,

2,6-Dimethyl-4-(2,3-dichlorphenyl)-pyridin-3,5-di-carbonsäure-(2-hydroxyethyl)-methyl-ester,

2,6-Dimethyl-4-(pyridyl-3)-pyridin-3,5-dicarbonsäure-(2-hydroxyethyl)-isopropyl-ester,

2,6-Dimethyl-4-(pyridyl-2)-pyridin-3,5-dicarbonsäure-(2-hydroxyethyl)-isopropyl-ester,

2,6-Dimethyl-4-(2-methylthio-pyridyl-3)-pyridin-3,5-dicarbonsäure-(2-hydroxyethyl)-isopropyl-ester,

2,6-Dimethyl-4-(2,1,3-benzoxadiazolyl-4)-pyridin-3,5-dicarbonsäure-(2-hydroxyethyl)-isopropyl-ester,

2,6-Dimethyl-4-(2,1,3-benzoxadiazolyl)-pyridin-3,5-dicarbonsäure-(2-hydroxyethyl)-methyl-ester,

6-Hydroxymethyl-2-methyl-4-(3-nitrophenyl)-pyridin-3,5-dicarbonsäure-3-(2-hydroxyethyl)-5-isopropyl-ester,

6-Hydroxymethyl-2-methyl-4-(3-nitrophenyl)-pyridin-3,5-dicarbonsäure-3-(2-cyanoethyl)-5-isopropyl-ester,

5,7-Dihydro-2-methyl-4-(2-nitrophenyl)-5-oxofuro-[3,4-b]pyridin-3-carbonsäure-(2-hydroxyethyl)-ester,

5,7-Dihydro-2-methyl-4-(2-trifluormethylphenyl)-5-oxo-furo[3,4-b]pyridin-3-carbonsäure-(2-cyanoethyl)-ester,

5,7-Dihydro-2-methyl-4-(pyridyl-3)-5-oxo-furo[3,4-b]-pyridin-3-carbonsäure-(2-acetoxyethyl)-ester.

Die genannten Verbindungen eignen sich besonders zur Behandlung von hypoxischen und/oder ischämischen Schädigungen vornehmlich des Zentralnervensystems sowie

sklerotisch, nekrotisch oder altersbedingten cerebralen Insuffizienzen und psychopathologischen Zuständen.

Die vorteilhaften Eigenschaften seien durch folgende Untersuchungen belegt:

**a) Hypoxieschutzwirkung im Modell der Hypoxie-induzierten retrograden Amnesie im Passive Avoidance Test**

(vgl. S.J. Sara & D. Lefevre, Psychopharmakologia, 25, 32-40, 1972).

In einem hell-dunkel Käfig werden Ratten mittels eines Elektroschocks trainiert, den dunklen Käfigteil zu vermeiden. Werden die Versuchstiere anschließend einer hypoxischen Atmosphäre (3,5 Vol.-% $O_2$) ausgesetzt, wird der Gedächtnisinhalt rückwirkend zerstört. Obengenannte Verbindungen antagonisieren die retrograde Amnesie vollständig.

**b) Erhöhung der Hypoxietoleranz**

Substanz- und placebobehandelte Mäuse werden in eine Kammer gesetzt, die solange von einem hypoxischen Gasgemisch (3,5 Vol.-% $O_2$) durchströmt wird, bis 85 % der Kontrolltiere tot sind. Obengenannte Verbindungen erhöhen die Anzahl der überlebenden Tiere signifikant.

**c) Hemmung des Abwehrverhaltens**

Isoliert gehaltene Mäuse zeigen auf elektrische Reizung hin "aggressive-defensive behaviour". Die erfindungsgemäßen Verbindungen, die ansonsten ohne allgemein sedierende Wirkung sind, hemmen dieses Verhalten vollständig.

Die genannten Verbindungen sind stark hypoxieschutzwirksam, obwohl sie weder Blutdruck noch Herzfrequenz beeinflussen und auch am isolierten Gefäßstreifen des Kaninchens nicht vasoaktiv sind. Die gezeigte psychotrope Wirkung ist besonders in der Gerontologie von zusätzlicher therapeutischer Bedeutung.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß-/Sesam-Öl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den o.g. Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,1 bis 5 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

**Herstellungsbeispiele**

**Beispiel 1**

2,6-Dimethyl-4-(3-nitrophenyl)-pyridin-3,5-dicarbonsäure-(2-hydroxyethyl)-isopropyl-ester

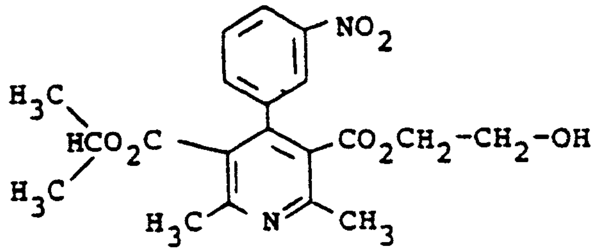

20 g (49,5 mMol) 1,4-Dihydro-2,6-dimethyl-4-(3-nitro-phenyl)-pyridin-3,5-dicarbonsäure-(2-hydroxyethyl)isopropyl-ester wurden in eine Mischung von 83 ml 96 %iger Salpetersäure in 660 ml Wasser eingetragen und 1 Stunde zum Sieden erhitzt. Anschließend wurde auf 5 bis 10°C abgekühlt und mit verdünnter Natronlauge schwach alkalisch gestellt. Das ausgefallene Öl wurde mit Methylenchlorid extrahiert, die Extrakte über Natriumsulfat getrocknet und im Vakuum eingeengt. Der ölige Rückstand wurde durch Verreiben mit Ether/Petrolether zur Kristallisation gebracht, abgesaugt und aus Methanol umkristallisiert.

Schmelzpunkt: 120°C; Ausbeute: 13,1 g (66 %).

**Beispiel 2**

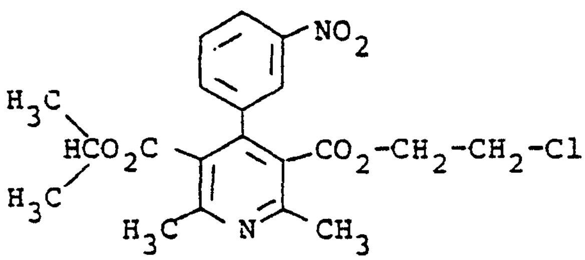

Analog Beispiel 1 wurde durch Umsetzung von 1,4-Dihydro-2,6-dimethyl-4-(3-nitrophenyl)-pyridin-3,5-dicarbonsäure -(2-chlorethyl)-isopropyl-ester mit Salpetersäure der 2,6-Dimethyl-4-(3-nitrophenyl)-pyridin-3,5-dicarbonsäure-(2-chlorethyl)-isopropyl-ester vom Fp: 80°C erhalten; Ausbeute: 69 % der Theorie.

**Beispiel 3**

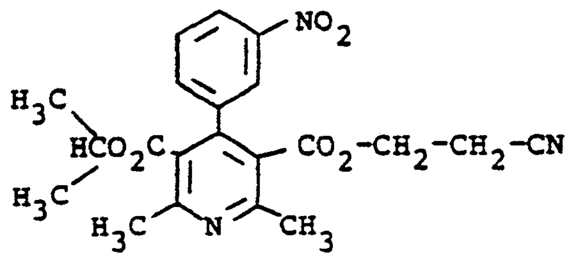

Analog Beispiel 1 wurde durch Umsetzung von 1,4-Dihydro-2,6-dimethyl-4-(3-nitrophenyl)-pyridin-3,5-dicarbonsäure -(2-cyanoethyl)-isopropyl-ester mit Salpetersäure der 2,6-Dimethyl-4-(3-nitrophenyl)-pyridin-3,5-dicarbonsäure-(2-cyanoethyl)-isopropyl-ester vom Fp: 93°C erhalten; Ausbeute: 71 % der Theorie.

**Beispiel 4**

2,6-Dimethyl-4-(3-nitrophenyl)-pyridin-3,5-dicarbonsäure -(2-acetoxyethyl)-isopropyl-ester

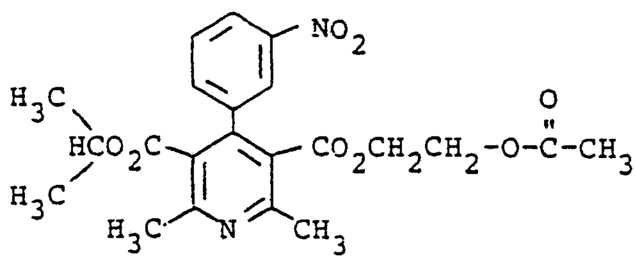

20,1 g (50 mmol) 2,6-Dimethyl-4-(3-nitrophenyl)-pyridin-3,5-dicarbonsäure-(2-acetoxyethyl)-isopropyl-ester (Beispiel 1) wurden in 75 ml Pyridin gelöst. Dazu wurden 5,9 g (75 mmol) Acetylchlorid zugegeben. Nach Beendigung der exothermen Reaktion wurde 3 Stunden bei Raumtemperatur gerührt, das Reaktionsgemisch in Wasser gegossen und mit $CH_2Cl_2$ extrahiert. Die organischen Extrakte wurden mit verdünnter Chlorwasserstoffsäure gewaschen und nach dem Trocknen über Natriumsulfat im Vakuum eingeengt. Das resultierende Öl kristallisierte durch, es wurde mit Petrolether verrührt, abgesaugt und getrocknet. Fp: 68°C; Ausbeute: 20,5 g (93 %).

**Beispiel 5**

2,6-Dimethyl-4-(3-nitrophenyl)-pyridin-3,5-dicarbon-säure-isopropyl-(2-methoxyethyl)-ester

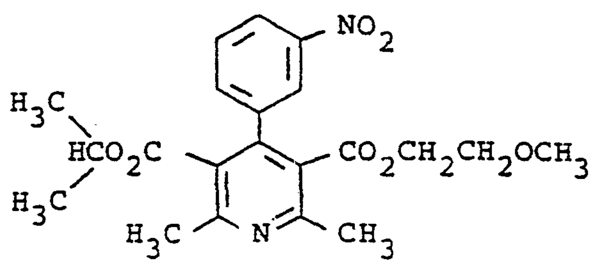

Zu einer siedenden Lösung von 41,8 g (100 mmol) 1,4-Dihydro-2,6-dimethyl-4-(3-nitrophenyl)-pyridin-3,5-dicarbonsäure-isopropyl-(2-methoxyethyl)-ester in 160 ml Eisessig wurden portionsweise 6,8 g Chrom(VI)-oxid zugegeben. Anschließend wurden weitere 30 Minuten unter Rückfluß erhitzt und nach dem Abkühlen in ammoniakalisches Eiswasser gegossen. Die Mischung wurde mit Chloroform extrahiert und die Extrakte nach Trocknen über Natriumsulfat im Vakuum eingeengt. Es resultierten 35,9 g (86 % der Theorie) eines Öls.

**Beispiel 6**

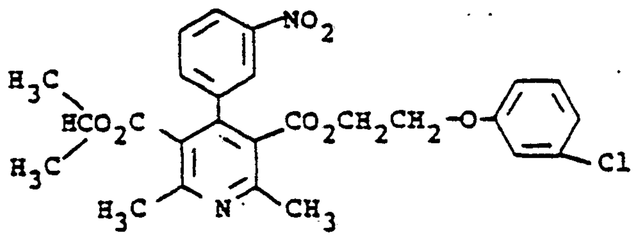

Analog Beispiel 1 wurde durch Umsetzung von 1,4-Dihydro-2,6-dimethyl-4-(3-nitrophenyl)-pyridin-3,5-dicarbonsäure-(2-(3-chlorphenoxy)-ethyl)-isopropyl-ester mit Salpetersäure der 2,6-Dimethyl-4-(3-nitrophenyl)-pyridin-3,5-dicarbonsäure-(2-(3-chlorphenoxy)-ethyl)-isopropylester vom Fp: 79° C erhalten; Ausbeute: 85 % der Theorie.

**Beispiel 7**

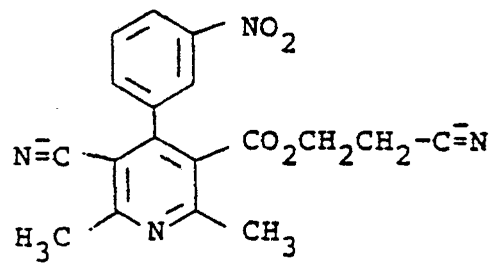

Analog Beispiel 1 wurde durch Umsetzung von 3-Cyano-1,4-dihydro-2,6-dimethyl-4-(3-nitrophenyl)-pyridin-5-carbonsäure-(2-cyanoethyl)-ester mit Salpetersäure der 3-Cyano-2,6-dimethyl-4-(3-nitrophenyl)-pyridin-5-carbonsäure-(2-cyanoethyl)-ester vom Fp: 142° C erhalten; Ausbeute: 69 % der Theorie.

**Beispiel 8**

3-Acetyl-2,6-dimethyl-4-(3-nitrophenyl)-pyridin-5-carbonsäure -(2-cyanoethyl)-ester

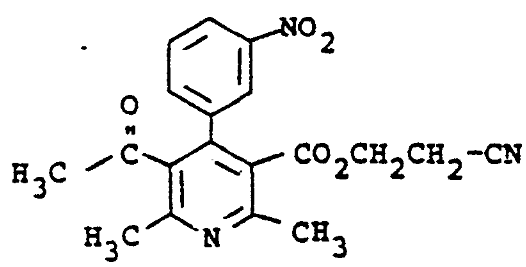

Eine Lösung von 10 g (27 mmol) 3-Acetyl-1,4-dihydro-2,6-dimethyl-4-(3-nitrophenyl)-pyridin-5-carbonsäure-(2-cyanoethyl)-ester in einem Elektrolyten von 5 g Lithiumperchlorat in 250 ml Acetonitril wurde an einer Platin-Anode elektrolysiert. Nach Durchgang von 2 Faraday-Äquivalenten wurde die Elektrolyse beendet, der Anolyt im Vakuum eingeengt, der Rückstand in einer Natriumbicarbonatlösung aufgenommen und mehrmals mit Methylenchlorid extrahiert. Die organischen Extrakte wurden nach Waschen mit Wasser über Natriumsulfat getrocknet und anschließend im Vakuum eingeengt. Das resultierende Öl kristallisierte durch, das feste Produkt wurde in Petrolether verrührt, abgesaugt und getrocknet, Fp: 85° C; Ausbeute: 7,5 g (75 %).

**Beispiel 9**

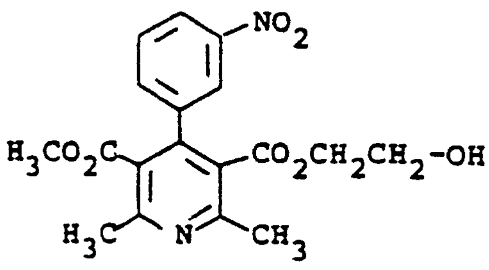

Analog Beispiel 1 wurde durch Umsetzung von 1,4-Dihydro-2,6-dimethyl-4-(3-nitrophenyl)-pyridin-3,5-dicarbonsäure-(2-hydroxyethyl)-methyl-ester mit Salpetersäure der 2,6-Dimethyl-4-(3-nitrophenyl)-pyridin-3,5-dicarbonsäure-(2-hydroxyethyl)-methyl-ester vom Fp: 115°C erhalten;

Ausbeute: 59 % der Theorie.

**Beispiel 10**

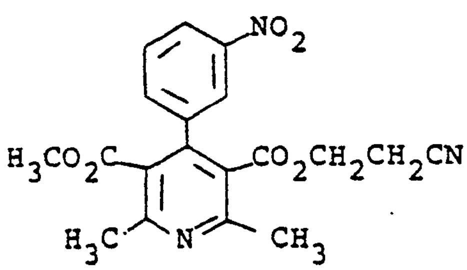

Analog Beispiel 1 wurde durch Umsetzung von 1,4-Dihydro-2,6-dimethyl-4-(3-nitrophenyl)-pyridin-3,5-dicarbon-säure-(2-cyanoethyl)-methyl-ester mit Salpetersäure der 2,6-Dimethyl-4-(3-nitrophenyl)-pyridin-3,5-dicarbonsäure -(2-cyanoethyl)-methyl-ester vom Fp: 98°C erhalten; Ausbeute: 65 % der Theorie. **Beispiel 11**

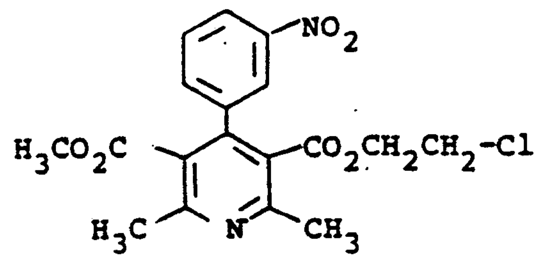

Analog Beispiel 1 wurde durch Umsetzung von 1,4-Dihydro-2,6-dimethyl-4-(3-nitrophenyl)-pyridin-3,5-dicarbonsäure-(2-chlorethyl)-methyl-ester mit Salpetersäure der 2,6-Dimethyl-4-(3-nitrophenyl)-pyridin-3,5-dicarbonsäure-(2-chlorethyl)-methyl-ester vom Fp: 60°C erhalten; Ausbeute: 55 % der Theorie.

**Beispiel 12**

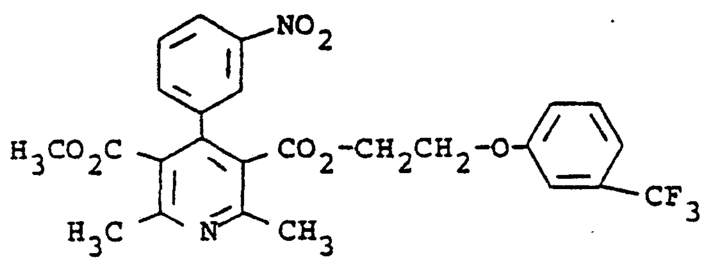

Analog Beispiel 1 wurde durch Umsetzung von 1,4-Dihydro-2,6-dimethyl-4-(3-nitrophenyl)-pyridin-3,5-dicarbonsäure-methyl-(2-(3-trifluormethylphenoxy)-ethyl)-ester mit Salpetersäure der 2,6-Dimethyl-4-(3-nitrophenyl)-pyridin -3,5-dicarbonsäure-methyl-(2-(3-trifluormethyl-phenoxy)-ethyl)-ester vom Fp: 88°C erhalten; Ausbeute: 89 % der Theorie.

**Beispiel 13**

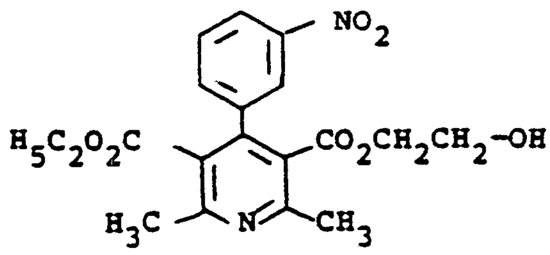

Analog Beispiel 1 wurde durch Umsetzung von 1,4-Dihydro-2,6-dimethyl-4-(3-nitrophenyl)-pyridin-3,5-dicarbonsäure-ethyl-(2-hydroxyethyl)-ester mit Salpetersäure der 2,6-Dimethyl-4-(3-nitrophenyl)-pyridin-3,5-dicarbonsäure-ethyl-(2-hydroxyethyl)-ester vom Fp: 88°C erhalten;
Ausbeute: 51 % der Theorie.

**Beispiel 14**

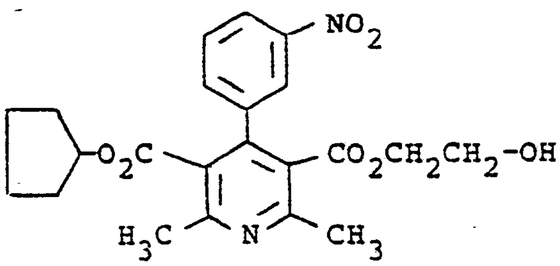

Analog Beispiel 1 wurde durch Umsetzung von 1,4-Dihydro-2,6-dimethyl-4-(3-nitrophenyl)-pyridin-3,5-dicarbonsäure -cyclopentyl-(2-hydroxyethyl)-ester mit Salpetersäure der 2,6-Dimethyl-4-(3-nitrophenyl)-pyridin-3,5-dicarbonsäure-cyclopentyl-(2-hydroxyethyl)-ester vom Fp: 112°C erhalten; Ausbeute: 63 % der Theorie.

**Beispiel 15**

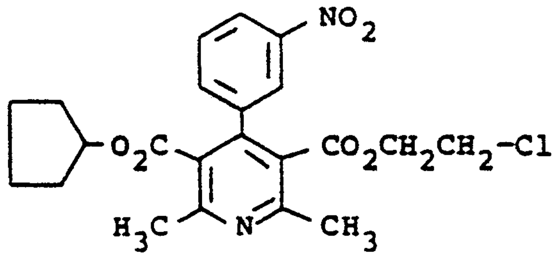

Analog Beispiel 1 wurde durch Umsetzung von 1 4 Dihydro-2,6-dimethyl-4-(3-nitrophenyl)-pyridin-3 5-dicarbonsäure (2-chlorethyl)-cyclopentyl-ester mit Salpetersäure der 2,6-Dimethyl-4-(3-nitrophenyl)-pyridin-3,5-dicarbonsäure-(2-chlorethyl)-cyclopentyl-ester vom Fp: 84°C erhalten;
Ausbeute: 71 % der Theorie.

**Beispiel 16**

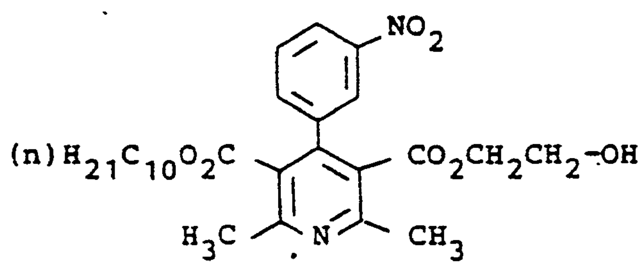

Analog Beispiel 1 wurde durch Umsetzung von 1,4-Dihydro-2,6-dimethyl-4-(3-nitrophenyl)-pyridin-3,5-dicarbonsäure-decyl-(2-hydroxyethyl)-ester mit Salpetersäure der 2,6-Dimethyl-4-(3-nitrophenyl)-pyridin-3,5-dicarbonsäure-decyl-(2-hydroxyethyl)-ester vom Fp: 60°C erhalten;
Ausbeute: 71 % der Theorie.

**Beispiel 17**

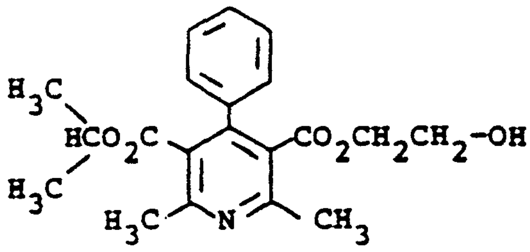

Analog Beispiel 1 wurde durch Umsetzung von 1,4-Dihydro-2,6-dimethyl-4-phenyl-pyridin-3,5-dicarbonsäure-(2-hy-droxyethyl)-isopropyl-ester mit Salpetersäure der 2,6-Dimethyl-4-phenyl-pyridin-3,5-dicarbonsäure-(2-hydroxy-ethyl)-isopropyl-ester vom Fp: 79°C erhalten; Ausbeute: 82 % der Theorie.

**Beispiel 18**

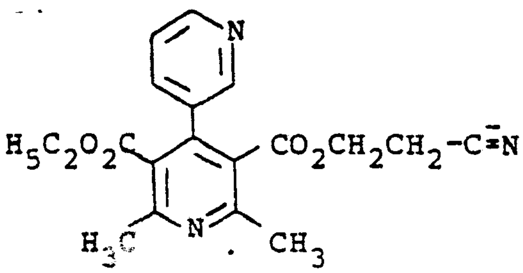

Analog Beispiel 1 wurde durch Umsetzung von 1,4-Dihydro-2,6-dimethyl-4-(pyridyl-3)-pyridin-3,5-dicarbonsäure-(2-cyanoethyl)-ethyl-ester mit Salpetersäure der 2,6-Dimethyl-4-(pyridyl-3)-pyridin-3,5-dicarbonsäure-(2-cyanoethyl)-ethyl-ester vom Fp: 90°C erhalten; Ausbeute: 52 % der Theorie.

**Beispiel 19**

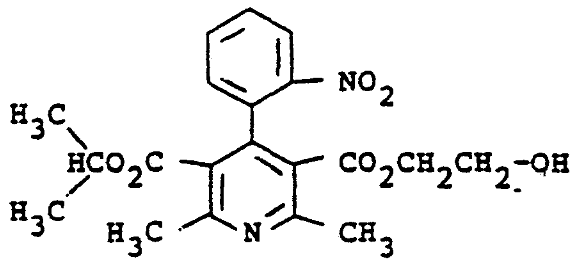

Analog Beispiel 1 wurde durch Umsetzung von 1,4-Dihydro-2,6-dimethyl-4-(2-nitrophenyl)-pyridin-3,5-dicarbon-säure-(2-hydroxyethyl)-isopropyl-ester mit Salpetersäure der 2,6-Dimethyl-4-(2-nitrophenyl)-pyridin-3,5-dicarbonsäure-(2-hydroxyethyl)-isopropyl-ester vom Fp: 78°C erhalten; Ausbeute: 83 % der Theorie.

**Beispiel 20**

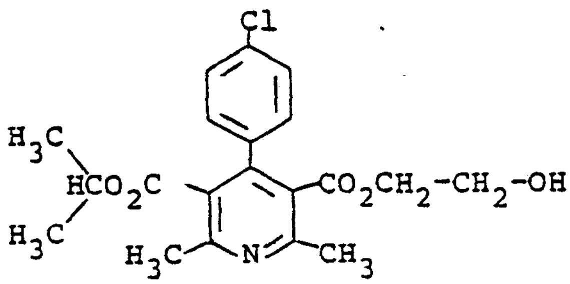

Analog Beispiel 1 wurde durch Umsetzung von 1,4-Dihydro-2,6-dimethyl-4-(4-chlorphenyl)-pyridin-3,5-dicarbon-säure-(2-hydroxyethyl)-isopropyl-ester mit Salpetersäure der 2,6-Dimethyl-4-(4-chlorphenyl)-pyridin-3,5-dicarbonsäure-(2-hydroxyethyl)-isopropylester vom Fp: 120°C erhalten; Ausbeute 88 % der Theorie.

**Beispiel 21**

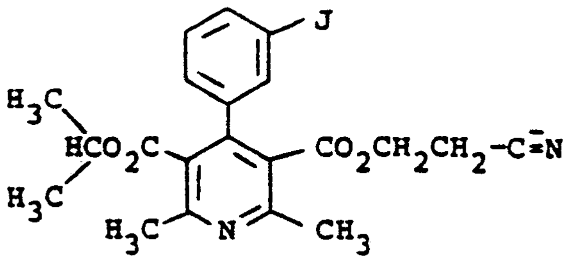

Analog Beispiel 1 wurde durch Umsetzung von 1,4-Dihydro-2,6-dimethyl-4-(3-jodphenyl)-pyridin-3,5-dicarbonsäure-(2-cyanoethyl)-isopropyl-ester mit Salpetersäure der 2,6-Dimethyl-4-(3-jodphenyl)-pyridin-3,5-dicarbonsäure-(2-cyanoethyl)-isopropyl-ester vom Fp: 69° C erhalten; Ausbeute: 75 % der Theorie.

**Beispiel 22**

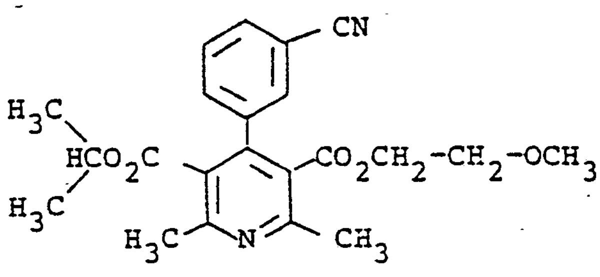

Analog Beispiel 1 wurde durch Umsetzung von 1,4-Dihydro-2,6-dimethyl-4-(3-cyanophenyl)-pyridin-3,5-dicarbonsäure -isopropyl-(2-methoxyethyl)-ester mit Salpetersäure der 2,6-Dimethyl-4-(3-cyanophenyl)-pyridin-3,5-dicarbonsäure-isopropyl-(2-methoxyethyl)-ester vom Fp: 50° C erhalten; Ausbeute: 73 % der Theorie.

**Beispiel 23**

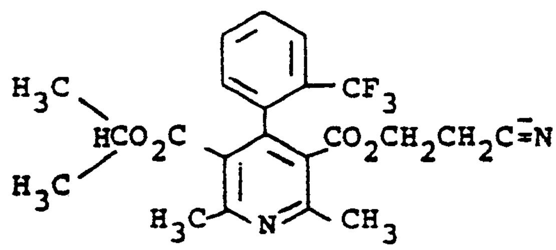

Analog Beispiel 1 wurde durch Umsetzung von 1,4-Dihydro-2,6-dimethyl-4-(2-trifluormethylphenyl)-pyridin-3,5-di-carbonsäure-(2-cyanoethyl)-methyl-ester mit Salpetersäure der 2,6-Dimethyl-4-(3-trifluormethylphenyl)-pyridin-3,5-dicarbonsäure-(2-cyanoethyl)-methyl-ester vom Fp: 74° C erhalten; Ausbeute: 85 % der Theorie.

**Beispiel 24**

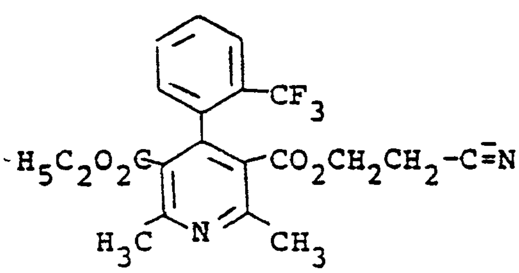

Analog Beispiel 1 wurde durch Umsetzung von 1,4-Dihydro-2,6-dimethyl-4-(2-trifluormethylphenyl)-pyridin-3,5-dicarbonsäure-(2-cyanoethyl)-ethyl-ester mit Salpetersäure der 2,6-Dimethyl-4-(2-trifluormethylphenyl)-pyri-din-3,5-dicarbonsäure-(2-cyanoethyl)-ethyl-ester vom Fp: 68° C erhalten; Ausbeute: 69 % der Theorie.

**Beispiel 25**

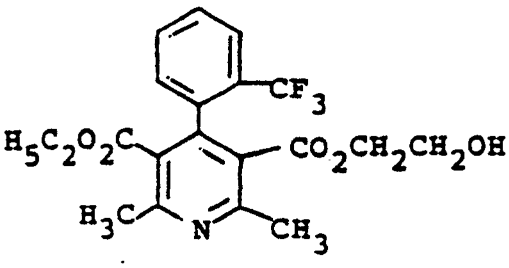

Analog Beispiel 1 wurde durch Umsetzung von 1,4-Dihydro-2,6-dimethyl-4-(2-trifluormethylphenyl)-pyridin-3-3,5-dicarbonsäure-ethyl-(2-hydroxyethyl)-ester mit Salpetersäure der 2,6-Dimethyl-4-(2-trifluormethylphenyl)-pyri-din-3,5-dicarbonsäureethyl-(2-hydroxyethyl)-ester vom Fp: 86° C erhalten; Ausbeute: 75 % der Theorie.

**Beispiel 26**

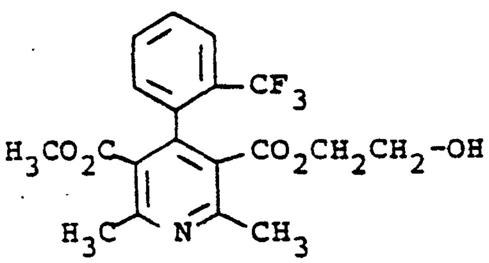

Analog Beispiel 1 wurde durch Umsetzung von 1,4-Dihydro-2,6-dimethyl-4-(2-trifluormethylphenyl)-pyridin-3,5-dicarbonsäure-(2-hydroxyethyl)-methyl-ester mit Salpetersäure der 2,6-Dimethyl-4-(2-trifluormethylphenyl)-pyridin-3,5-dicarbonsäure-(2-hydroxyethyl)-methylester vom Fp: 75° C erhalten; Ausbeute: 71 % der Theorie.

**Beispiel 27**

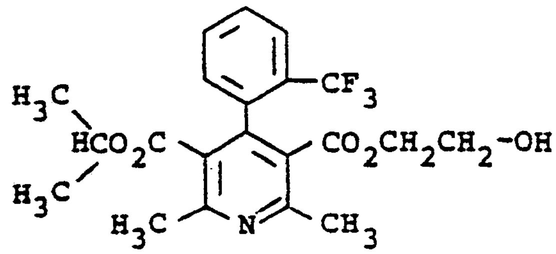

Analog Beispiel 1 wurde durch Umsetzung von 1,4-Dihydro-2,6-dimethyl-4-(2-trifluormethylphenyl)-pyridin-3,5-dicarbonsäure-(2-hydroxyethyl)-isopropyl-ester mit Salpetersäure der 2,6-Dimethyl-4-(2-trifluormethylphenyl)-pyridin-3,5-dicarbonsäure-(2-hydroxyethyl)-isopropylester vom Fp: 83° C erhalten; Ausbeute: 51 % der Theorie.

**Beispiel 28**

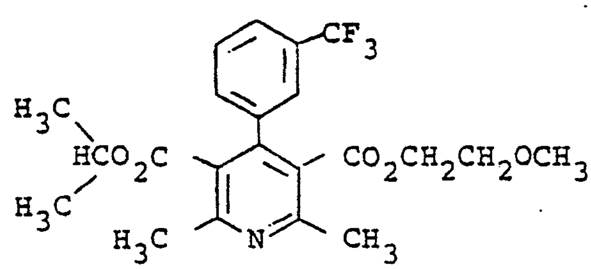

Analog Beispiel 1 wurde durch Umsetzung von 1,4-Dihydro-2,6-dimethyl-4-(3-trifluormethylphenyl)-pyridin-3,5-dicarbonsäure-isopropyl-(2-methoxyethyl)-ester mit Salpetersäure der 2,6-Dimethyl-4-(3-trifluormethylphenyl)-pyridin-3,5-dicarbonsäure-isopropyl-(2-methoxyethyl)-ester vom Fp: 51° C erhalten; Ausbeute: 65 % der Theorie.

**Beispiel 29**

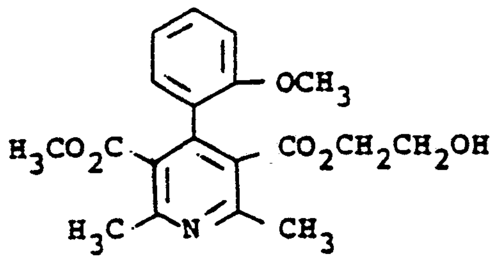

Analog Beispiel 1 wurde durch Umsetzung von 1,4-Dihydro-2,6-dimethyl-4-(2-methoxyphenyl)-pyridin-3,5-dicarbonsäure -(2-hydroxyethyl)-methyl-ester mit Salpetersäure der 2,6-Dimethyl-4-(2-methoxyphenyl)-pyridin-3,5-dicar-bonsäure-(2-hydroxyethyl)-methyl-ester vom Fp: 90° C erhalten; Ausbeute: 84 % der Theorie.

**Beispiel 30**

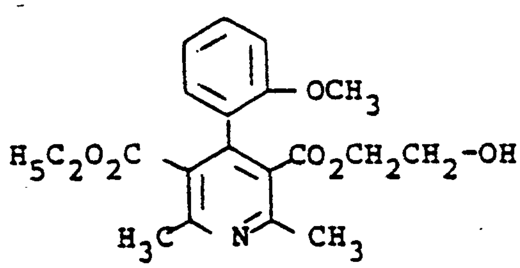

Analog Beispiel 1 wurde durch Umsetzung von 1,4-Dihydro-2,6-dimethyl-4-(2-methoxyphenyl)-pyridin-3,5-dicarbonsäure -ethyl-(2-hydroxyethyl)-ester mit Salpetersäure der 2,6-Dimethyl-4-(2-methoxyphenyl)-pyridin-3,5-dicarbon-säureethyl-(2-hydroxyethyl)-ester vom Fp: 95° C erhalten; Ausbeute: 71 % der Theorie.

**Beispiel 31**

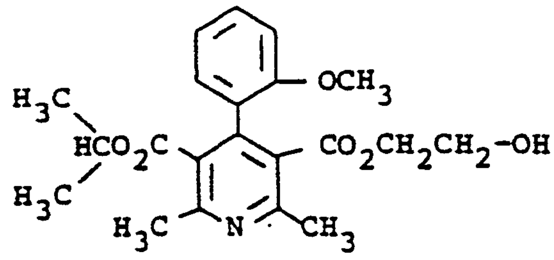

Analog Beispiel 1 wurde durch Umsetzung von 1,4-Dihydro-2,6-dimethyl-4-(2-methoxyphenyl)-pyridin-3,5-dicarbonsäure -(2-hydroxyethyl)-isopropyl-ester mit Salpetersäure der 2,6-Dimethyl-4-(2-methoxyphenyl)-pyridin-3,5-dicar-bonsäure-(2-hydroxyethyl)-isopropyl-ester vom Fp: 68° C erhalten; Ausbeute: 85 % der Theorie.

**Beispiel 32**

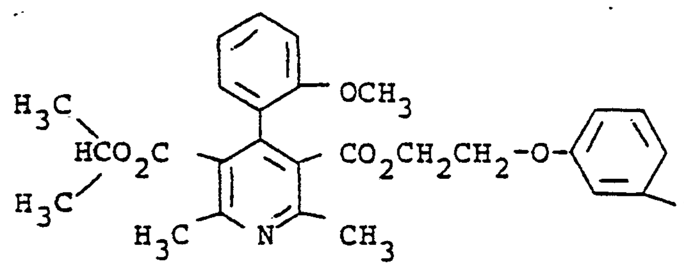

Analog Beispiel 1 wurde durch Umsetzung von 1,4-Dihydro-2,6-dimethyl-4-(2-methoxyphenyl)-pyridin-3,5-dicarbonsäure -isopropyl-(2-(3-trifluormethylphenoxy)-ethyl)ester mit Salpetersäure der 2,6-Dimethyl-4-(2-methoxyphenyl)-pyridin-3,5-dicarbonsäure-isopropyl-(2-(3-trifluormethylphenoxy)-ethyl)-ester vom Fp: 80° C erhalten;

Ausbeute: 65 % der Theorie.

**Beispiel 33**

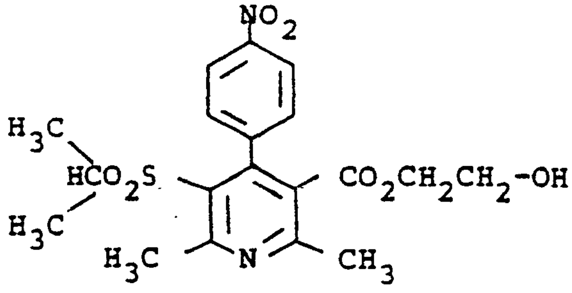

Analog Beispiel 1 wurde durch Umsetzung von 1,4-Dihydro-2,6-dimethyl-4-(4-nitrophenyl)-pyridin-3,5-dicarbonsäure-(2-hydroxyethyl)-isopropyl-ester mit Salpetersäure der 2,6-Dimethyl-4-(4-nitrophenyl)-pyridin-3,5-dicarbonsäure-(2-hydroxyethyl)-isopropyl-ester vom Fp: 145° C erhalten; Ausbeute: 62 % der Theorie.

**Beispiel 34**

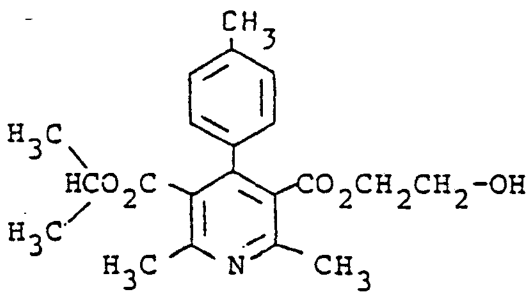

Analog Beispiel 1 wurde durch Umsetzung von 1,4-Dihydro-2,6-dimethyl-4-(4-methylphenyl)-pyridin-3,5-dicarbonsäure -(2-hydroxyethyl)-isopropyl-ester mit Salpetersäure der 2,6-Dimethyl-4-(4-methylphenyl)-pyridin-3,5-dicarbonsäure-(2-hydroxyethyl)-isopropyl-ester vom Fp: 72° C erhalten; Ausbeute: 56 % der Theorie.

**Beispiel 35**

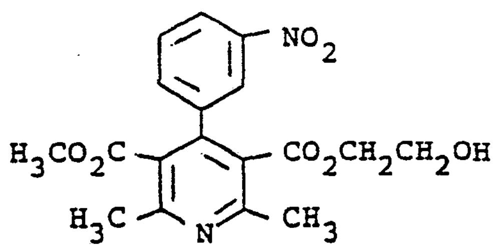

Analog Beispiel 1 wurde durch Umsetzung von 1,4-Dihydro-2,6-dimethyl-3-methylsulfonyl)-4-(3-nitrophenyl)-pyridin -5-carbonsäure-(2-hydroxyethyl)-ester mit Salpetersäure der 2,6-Dimethyl-3-methylsulfonyl-4-(3-nitrophe-nyl)-pyridin-5-carbonsäure-(2-hydroxyethyl)-ester vom Fp: 120° C erhalten; Ausbeute: 53 % der Theorie.

**Beispiel 36**

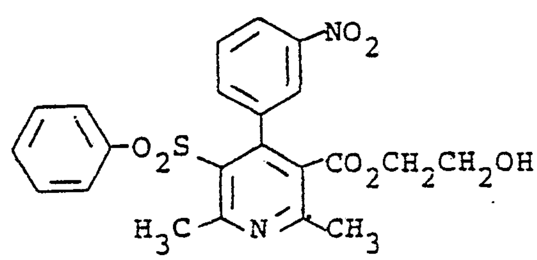

Analog Beispiel 1 wurde durch Umsetzung von 1,4-Dihydro-2,6-dimethyl-4-(3-nitrophenyl)-3-phenylsulfonyl-pyridin-5-carbonsäure-(2-hydroxyethyl)-ester mit Salpetersäure der 2,6-Dimethyl-4-(3-nitrophenyl)-3-phenylsulfonyl-pyridin-5-carbonsäure-(2-hydroxyethyl)-ester vom Fp: 150° C erhalten; Ausbeute: 41 % der Theorie.

**Beispiel 37**

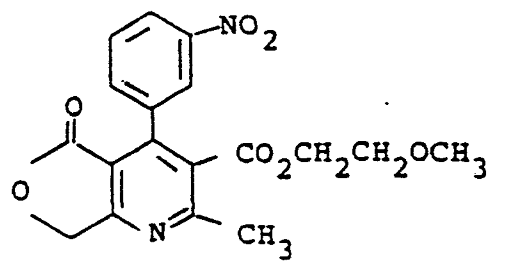

Analog Beispiel 9 wurde durch Umsetzung von 1,4,5,7-Tetrahydro-2-methyl-4-(3-nitrophenyl)-5-oxo-furo[3,4-b]-pyridin-3-carbonsäure-(2-methoxyethyl)-ester mit Chrom(VI)-oxid in Eisessig der 5,7-Dihydro-2-methyl-4-(3-nitrophenyl)-5-oxo-furo/3,4-b/pyridin-3-carbonsäure-(2-methoxyethyl)-ester vom Fp: 114°C erhalten; Ausbeute: 75 % der Theorie.

**Beispiel 38**

Kalium-Salz des 2-Hydroxymethyl-6-methyl-4-(3-nitro-phenyl)-pyridin-3,5-dicarbonsäure-5-(2-hydroxyethyl)esters

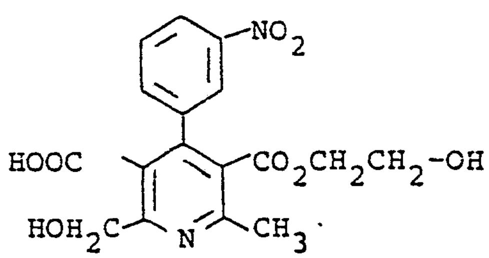

24 g (50 mmol) 2-Acetoxymethyl-6-methyl-4-(3-nitrophenyl)-pyridin-3,5-dicarbonsäure-5-(2-acetoxyethyl)-3-ethyl-ester wurden in 120 ml 1,2-Dimethoxyethan gelöst und nach vorsichtiger Zugabe einer Lösung von 6,2 g Kaliumhydroxid in 120 ml Wasser 2 Stunden bei Raumtemperatur gerührt. Anschließend wurde die Mischung mehrmals mit Methylenchlorid extrahiert, die wäßrige Phase im Vakuum zur Trockne eingedampft und der Rückstand aus Isopropanol umkristallisiert, Fp: 223°C; Ausbeute: 3,6 g (17 % der Theorie).

**Beispiel 39**

5,7-Dihydro-2-methyl-4-(3-nitrophenyl)-5-oxo-furo[3,4-b]-pyridin-3-carbonsäure-(2-hydroxyethyl)-ester

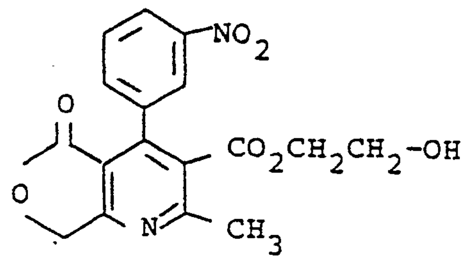

4 g des Kalium-Salzes des 2-Hydroxymethyl-6-methyl-4-(3-nitrophenyl)-pyridin-3,5-dicarbonsäure-5-(2-hydroxy-ethyl)-esters (Beispiel 38) wurden in 4 ml Wasser gelöst und mit konzentrierter Chlorwasserstoffsäure angesäuert. Nach Stehen über Nacht bei Raumtemperatur wurde mit Wasser verdünnt, das ausgefallene öl mit Methylenchlorid extrahiert, die organischen Extrakte nach Trocknen über Natriumsulfat im Vakuum eingeengt und der Rückstand aus Isopropanol umkristallisiert, Fp: 130°C; Ausbeute: 2,3 g (64 % der Theorie).

**Patentansprüche**

1. Pyridincarbonsäureester der allgemeinen Formel

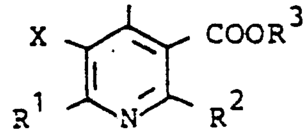

(I)

in welcher

R für einen Phenyl- oder Pyridylrest steht, wobei der Phenylrest gegebenenfalls 1- oder 2-fach substituiert ist durch Nitro, Cyano, Trifluormethyl, Fluor, Chlor, Brom Alkyl, Alkoxy, Alkylmercapto mit jeweils 1 bis 4 Kohlenstoffatomen in den Alkyl- und Alkoxyresten, oder für einen Benzoxadiazolylrest

steht,

$R^1$ für Wasserstoff oder einen Alkylrest mit 1 bis 4 Kohlenstoffatomen, die Phenylgruppe, die Benzylgruppe, die Acetoxymethylgruppe, die 2-. Acetoxyethylgruppe oder eine Hydroxyalkylgruppe mit 1 bis 4 Kohlenstoffatomen steht, oder

$R^1$ zusammem mit X einen 5- bis 7-gliedrigen Ring bilden, der als Ringglied eine Carbonylgruppe und gegebenenfalls ein Sauerstoff- oder Stickstoffatom enthält, wobei der Stickstoff gegebenenfalls noch einen Wasserstoff oder einen niederen Alkylrest mit 1 bis 4 C-Atomen trägt,

$R^2$ für Wasserstoff oder für einen geradkettigen oder verzeigten Alkylrest mit 1 bis 4 Kohlenstoffatomenn, Phenyl, Benzyl. Acetoxymethyl 2-Acetoxyethyl oder Hydroxyalkyl mit 1 bis 4 Kohlenstoffatomen steht,

X für eine Nitrilgruppe steht

oder

für den Rest $COR^4$ steht, wobei $R^4$ einen geradkettigen oder verzweigten Alkylrest mit 1 bis 4 Kohlenstoffatomen, Phenyl oder Benzyl darstellt

oder

für die Gruppe $COOR^5$ steht, wobei $R^5$ einen geradkettigen, verzweigten oder cyclischen gesättigten oder ungesättigten Kohlenwasserstoffrest mit bis zu 10 Kohlenstoffatomen darstellt, der gegebenenfalls durch ein Sauerstoffatom in der Kette unterbrochen ist und/ oder der gegebenenfalls substituiert ist durch Fluor, Chlor, Brom, Cyano, Hydroxy, Acetoxy, α-, β- oder γ-Pyridyl, Phenyl, Phenoxy, wobei die Arylgruppen ihrerseits durch Fluor, Chlor, Nitro, Trifluormethyl, Alkyl, Alkoxy mit jeweils 1 bis 4 Kohlenstoffatomen oder Amino substituiert ist, wobei die Aminogruppe ihrerseits gegebenenfalls durch zwei gleiche oder verschiedene Substituenten aus der Gruppe Alkyl mit 1 bis 4 Kohlenstoffatomen, Phenyl oder Benzyl substituiert ist oder

wobei $R^5$ auch Wasserstoff sein kann, wenn $R^1$ eine Hydroxyalkyl- oder Acyloxyalkylgruppe darstellt,

oder

für die Gruppe $SO_2R^6$ steht, wobei $R^6$ einen Alkylrest mit 1 bis 4 Kohlenstoffatomen oder Phenyl darstellt, welches gegebenenfalls durch Fluor, Chlor, Trifluormethyl, Alkyl oder Alkoxy mit jeweils 1 bis 2 Kohlenstoffatomen substituiert ist,

$R^3$ immer verschieden von $R^5$ ist und für einen geradkettigen Kohlenwasserstoffrest mit bis zu 6 Kohlenstoffatomen steht, der durch ein Sauerstoffatom in der Kette unterbrochen ist und/oder der substituiert ist durch Fluor, Chlor, Brom, Cyano, Hydroxy, Acetoxy, Dialkylamino mit bis zu 4 Kohlenstoffatomen je Alkylgruppe, α-, β- oder γ -Pyridyl, Phenyl oder Phenoxy, wobei die Arylgruppen ihrerseits durch Trifluormethyl, Fluor, Chlor, Nitro, Alkyl oder Alkoxy mit jeweils 1 bis 4 Kohlenstoffatomen substituiert sein können,

sowie ihre pharmazeutisch unbedenklichen Salze.

2. 5,7-Dihydro-2-methyl-4-(3-nitrophenyl)-5-oxo-furo 3,4-b -pyridin-3-carbonsäure-(2-hydroxyethyl)-ester.

3. Verbindungen gemäß den Ansprüchen 1 und 2 zur Verwendung bei der Bekämpfung von Erkrankungen.

4. Verbindungen gemäß den Ansprüchen 1 und 2 zur Verwendung bei der Bekämpfung von Erkrankungen, die auf einer Ischämie und/oder Hypoxy beruhen.

5. Arzneimittel enthaltend mindestens eine Verbindung gemäß den Ansprüchen 1 oder 2.

6. Arzneimittel mit Hypoxieschutzwirkung enthaltend mindestens eine Verbindung gemäß den Ansprüchen 1 oder 2.

7. Verfahren zur Herstellung von Pyridincarbonsäureestern der allgemeinen Formel I

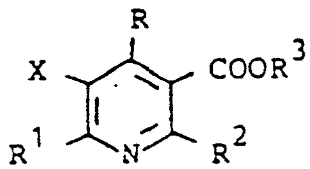

(I)

in welcher

R für einen Phenyl- oder Pyridylrest steht, wobei der Phenylrest gegebenenfalls 1- oder 2-fach substituiert ist durch Nitro, Cyano, Trifluormethyl, Fluor, Chlor, Brom, Alkyl, Alkoxy, Alkylmercapto mit jeweils 1 bis 4 Kohlenstoffatomen in den Alkyl- und Alkoxyresten, oder für einen Benzoxadiazolylrest steht,

$R^1$ für Wasserstoff oder einen Alkylrest mit 1 bis 4 Kohlenstoffatomen, die Phenylgruppe, die Benzylgruppe, die Acetoxymethylgruppe, die 2-Acetoxyethylgruppe oder eine Hydroxyalkylgruppe mit 1 bis 4 Kohlenstoffatomen steht,

oder

$R^1$ zusammen mit X einen 5- bis 7-gliedrigen Ring bilden, der als Ringglied eine Carbonylgruppe und gegebenenfalls ein Sauerstoff- oder Stickstoffatom enthält, wobei der Stickstoff gegebenenfalls noch einen Wasserstoff oder einen niederen Alkylrest mit 1 bis 4 C-Atomen trägt,

$R^2$ für Wasserstoff oder für einen geradkettigen oder verzweigten Alkylrest mit 1 bis 4 Kohlenstoffatomen, Phenyl, Benzyl, Acetoxymethyl, 2-Acetoxyethyl oder Hydroxyalkyl mit 1 bis 4 Kohlenstoffatomen steht,

X für eine Nitrilgruppe steht

oder

für den Rest $COR^4$ steht, wobei $R^4$ einen geradkettigen oder verzweigten Alkylrest mit 1 bis 4 Kohlenstoffatomen, Phenyl oder Benzyl darstellt

oder

für die gruppe $COOR^5$ steht, wobei $R^5$ einen geradkettigen, verzweigten oder cyclischen gesättigten oder ungesättigten Kohlenwasserstoffrest mit bis zu 10 Kohlenstoffatomen darstellt, der gegebenenfalls durch ein Sauerstoffatom in der Kette

unterbrochen ist und/oder der gegebenenfalls substituiert ist durch Fluor, Chlor, Brom, Cyano, Hydroxy, Acetoxy, α-, β- oder γ -Pyridyl, Phenyl, Phenoxy, wobei die Arylgruppen ihrerseits durch Fluor,: Chlor, Nitro, Trifluormethyl, Alkyl, Alkoxy; mit jeweils 1 bis 4 Kohlenstoffatomen oder Amino substituiert ist, wobei die Aminogruppe ihrerseits gegebenenfalls durch zwei gleiche oder verschiedene Substituenten aus der Gruppe Alkyl mit 1 bis 4 Kohlenstoffatomen, Phenyl oder Benzyl substituiert ist oder wobei $R^5$ auch Wasserstoff sein kann, wenn $R^1$ eine Hydroxyalkyl- oder Acyloxyalkylgruppe darstellt,

oder

für die Gruppe $SO_2R^6$ steht, wobei $R^6$ einen Alkylrest mit 1 bis 4 Kohlenstoffatomen oder Phenyl darstellt, welches gegebenenfalls durch Fluor, Chlor, Trifluormethyl, Alkyl oder Alkoxy mit jeweils 1 bis 2 Kohlenstoffatomen substituiert ist,

$R^3$ immer verschieden von $R^5$ ist und für einen geradkettigen Kohlenwasserstoffrest mit bis zu 6 Kohlenstoffatomen steht, der durch ein Sauerstoffatom in der Kette unterbrochen ist und/oder der substituiert ist durch Fluor, Chlor, Brom, Cyano, Hydroxy, Acetoxy, Dialkylamino mit bis zu 4 Kohlenstoffatomen je Alkylgruppe, α-, β- oder δ -Pyridyl, phenyl oder phenoxy, wobei die Arylgruppen ihrerseits durch Trifluormethyl, Fluor, Chlor, Nitro, Alkyl oder Alkoxy mit jeweils 1 bis 4 Kohlenstoffatomen substituiert sein können,

sowie ihrer pharmazeutisch unbedenklichen Salze,

dadurch gekennzeichnet, daß man

A) 1,4-Dihydropyridinderivate der allgemeinen Formel (II)

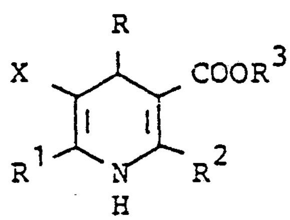

(II)

in welcher

R, $R^1$, $R^2$, $R^3$ und X die oben angegebene Bedeutung haben,

mit oxidierenden (dehydrierenden) Agentien gegebenenfalls in Gegenwart inerter Lösungsmittel bei Temperaturen zwischen 0 und 200°C zur Reaktion bringt,

oder

B) Pyridincarbonsäuren der allgemeinen Formel (III)

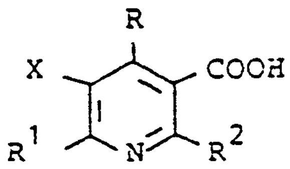

(III)

in welcher

R, $R^1$, $R^2$ und X die oben angegebene Bedeutung haben,

gegebenenfalls nach Aktivierung der Carboxylgruppe entsprechend literaturbekannten Methoden zu Carbonsäureestern der allgemeinen Formel (I) verestert,

oder

C) für den Fall, daß $R^1$ und/oder $R^2$ in der allgemeinen Formel (I) eine Hydroxyalkylgruppe darstellen, die entsprechenden Mono- bzw. Bisacetoxyalkylpyridine den Bedingungen einer milden und selektiven Hydrolyse unterwirft, oder

D) für den Fall, daß X in der Formel (I) Carboxyl bedeutet und $R^1$ Hydroxyalkyl bedeutet, Verbindungen der Formel (IV)

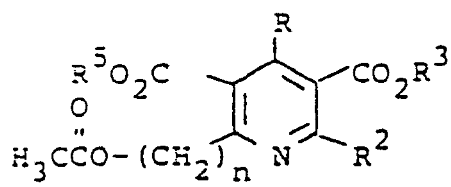

(IV)

in welcher

R, $R^2$, $R^3$, $R^5$, und n die oben angegebene Bedeutung haben,

in Gegenwart von Alkali zu Verbindungen der Formel (V) verseift,

oder

E) für den Fall, daß $R^1$ mit X in der allgemeinen Formel (I) einen Lactonring bildet, Hydroxyalkylpyridincarbonsäuren der allgemeinen Formel (V)

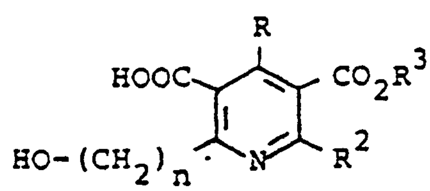

(V)

in welcher

R, $R^2$ und $R^3$ die oben angegebene Bedeutung haben und

n eine Zahl von 1 bis 4 darstellt,

unter dem Einfluß von Protonen nach üblichen Methoden zu Verbindungen der Formel (VI)

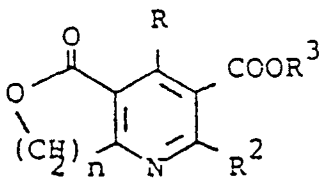

(VI)

in welcher

R, $R^2$, $R^3$ und n die oben angegebene Bedeutung haben,

cyclisiert.

8. Verfahren zur Herstellung von Arzneimitteln, dadurch gekennzeichnet, daß man Verbindungen gemäß den Ansprüchen 1 und 2, gegebenenfalls unter Verwendung von üblichen Hilfs- und Trägerstoffen in eine geeignete Applikationsform überführt.

## Claims

Pyridinecarboxylic acid esters of the general formula I

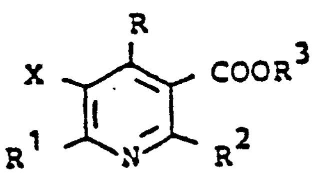

(I)

in which

R represents a phenyl or pyridyl radical, the phenyl radical optionally being substituted once or twice by nitro, cyano, trifluoromethyl, fluorine, chlorine, bromine alkyl, alkoxy or alkylmercapto, each having 1 to 4 carbon atoms in the alkyl and alkoxy radicals,or represent a benzoxadiazolyl radical, $R^1$ represents hydrogen or an alkyl radical having 1 to 4 carbon atoms, the phenyl group, the benzyl group, the acetoxymethyl group, the 2-acetoxyethyl group or a hydroxyalkyl group having 1 to 4 carbon atoms, or

$R^1$, together with X, forms a 5- to 7-membered ring, which contains as ring member a carbonyl group and optionally an oxygen or nitrogen atom, the nitrogen optionally also carrying a hydrogen atom or a lower alkyl radical having 1 to 4 C atoms,

$R^2$ represents hydrogen or a straightchain or branched alkyl radical having 1 to 4 carbon atoms, phenyl, benzyl, acetoxymethyl, 2-acetoxyethyl or hydroxyalkyl having 1 to 4 carbon atoms, X represents a nitrile group or represents the radical $COR^4$, $R^4$ representing a straight-chain or branched alkyl radical having 1 to 4 carbon atoms, phenyl or benzyl, or represents the group $COOR^5$, $R^5$ representing a straight-chain, branched or cyclic, saturated or unsaturated hydrocarbon radical having up to 10 carbon atoms, which is optionally interrupted by an oxygen atom in the chain and/or is optionally substituted by fluorine, chlorine, bromine, cyano, hydroxyl, acetoxy, α-, β- or γ-pyridyl, phenyl or phenoxy, the aryl groups in turn being substituted by fluorine, chlorine, nitro trifluoromethyl, alkyl, alkoxy, each having 1 to 4 carbon atoms, or amino, the amino group in turn optionally being substituted by two identical or different substituents from the group comprising alkyl having 1 to 4 carbon atoms, phenyl or benzyl, or it being possible for $R^5$ also to be hydrogen when $R^1$ represents a hydroxyalkyl or acyloxyalkyl group, or represents the group $SO_2R^6$, $R^6$ representing an alkyl radical having 1 to 4 carbon atoms or phenyl, which is optionally substituted by fluorine, chlorine, trifluoromethyl, alkyl or alkoxy, each having 1 to 2 carbon atoms, $R^3$ is always different from $R^5$ and represents a straight-chain hydrocarbon radical having up to 6 carbon atoms, which is interrupted by an oxygen atom in the chain and/or is substituted by fluorine, chlorine, bromine, cyano, hydroxyl, acetoxy, dialkylamino having up to 4 carbon atoms per alkyl group, α-, β- or γ-pyridyl, phenyl or phenoxy, it being possible for the aryl groups in turn to be substituted by trifluoromethyl, fluorine, chlorine, nitro, alkyl or alkoxy, each having 1 to 4 carbon atoms.

and pharmaceutically harmless salts thereof.

2. 5,7-Dihydro-2-methyl-4-(3-nitrophenyl)-5-oxo-furo 3,4-b -pyridine-3-carboxylic acid (2-hydroxyethyl) ester.

3. Compounds according to Claims 1 amd 2 for use in combating diseases.

4. Compounds according to Claims 1 and 2 for use in combating diseases caused by ischemia and/or hypoxia.

5. Medicaments containing at least one compound according to Claims 1 or 2.

6. Medicaments having protective action against hypoxia, containing at least one compound according to Claims 1 or 2.

7. Process for the preparation of pyridinecarboxylic acid esters of the general formula (I)

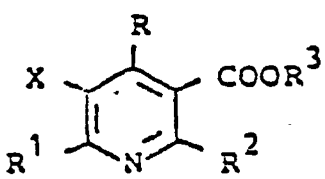

(I)

in which

R represents a phenyl or pyridyl radical, the phenyl radical optionally being substituted once or twice by nitro, cyano, trifluoromethyl, fluorine, chlorine, bromine, alkyl, alkoxy or alkylmercapto, each having 1 to 4 carbon atoms in the alkyl and alkoxy radicals, or represents a benzoxadiazolyl radical, $R^1$ represents hydrogen or an alkyl radical having 1 to 4 carbon atoms, the phenyl group, the benzyl group, the acetoxymethyl group, the 2-acetoxyethyl group or a hydroxyalkyl group having 1 to 4 carbon atoms, or

$R^1$, together with X, forms a 5- to 7-membered ring, which contains as ring member a carbonyl group and optionally an oxygen or nitrogen atom, the nitrogen optionally also carrying a hydrogen atom or a lower alkyl radical having 1 to 4 C atoms,

$R^2$ represents hydrogen or a straightchain or branched alkyl radical having 1 to 4 carbon atoms, phenyl, benzyl, acetoxymethyl, 2-acetoxyethyl or hydroxyalkyl having 1 to 4 carbon atoms, X represents a nitrile group or represents the radical $COR^4$, $R^4$ representing a straight-chain or branched alkyl radical having 1 to 4 carbon atoms, phenyl or benzyl, or represents the group $COOR^5$, $R^5$ representing a straight-chain, branched or cyclic, saturated or unsaturated hydrocarbon radical having up to 10 carbon atoms, which is optionally interrupted by an oxygen atom in the chain and/or is optionally substituted by fluorine, chlorine, bromine, cyano, hydroxyl, acetoxy, α-, β- or γ-pyridyl, phenyl or phenoxy, the aryl groups in turn being substituted by fluorine, chlorine, nitro, trifluoromethyl, alkyl, alkoxy, each having 1 to 4 carbon atoms, or amino, the amino group in turn optionally being substituted by two identical or different substituents from the group comprising alkyl having 1 to 4 carbon atoms, phenyl or benzyl, or it being possible for $R^5$ also to be hydrogen when $R^1$ represents a hydroxyalkyl or acyloxyalkyl group, or represents the group $SO_2R^6$, $R^6$ representing an alkyl radical having 1 to 4 carbon atoms or phenyl, which is optionally substituted by fluorine, chlorine, trifluoromethyl, alkyl or alkoxy, each having 1 to 2 carbon atoms, $R^3$ is always different from $R^5$ and represents a straight-chain hydrocarbon radical having up to 6 carbon atoms, which is interrupted by an oxygen atom in the chain and/or is substituted by fluorine, chlorine, bromine, cyano, hydroxyl, acetoxy, dialkylamino having up to 4 carbon atoms per alkyl group, α -, β- or γ-pyridyl, phenyl or phenoxy, it being possible for the aryl groups in turn to be substituted by trifluoromethyl, fluorine, chlorine, nitro, alkyl or alkoxy, each having 1 to 4 carbon atoms.

and pharmaceutically harmless salts thereof, characterised in that

A) 1,4-dihydropyridine derivatives of the general formula (II)

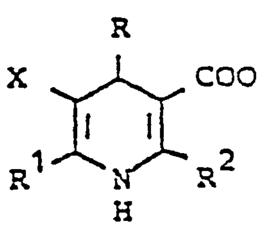

(II)

in which

R, $R^1$, $R^2$, $R^3$ and X have the meaning indicated above,

are reacted with oxidising (dehydrogenating) agents, optionally in the presence of inert solvents, at temperatures between 0 and 200°C, or

B) pyridinecarboxylic acids of the general formula (III)

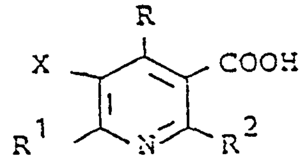

(III)

in which

R, $R^1$, $R^2$ and X have the meaning indicated above, are esterified optionally after activation of the carboxyl group in accordance with methods known from the literature, to give carboxylic esters of the general formula (I), or C) in the case where $R^1$ and/or $R^2$ in the general formula (I) represent a hydroxyalkylgroup, the corresponding monoacetoxyalkylpyridines or bisacetoxyalkylpyridines are subjected to the conditions of mild and selective hydrolysis, or

D) in the case where X in the formula (I) denotes carboxyl and $R^1$ denotes hydroxyalkyl, compounds of the formula (IV)

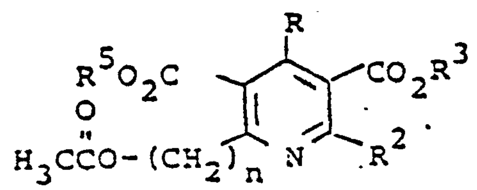

(IV)

in which

R, $R^2$, $R^3$, $R^5$ and n have the meaning indicated above,

are hydrolysed in the presence of alkali to give compounds of the formula (V), or

E) in the case where $R^1$ and X in the general formula (I) form a lactone ring, hydroxyalkylpyridinecarboxylic acids of the general formula (V)

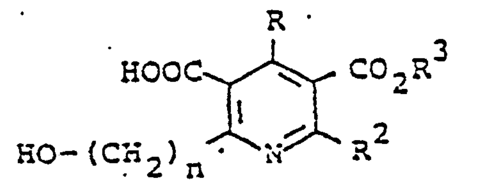

(V)

in which

R, $R^2$ and $R^3$ have the meaning indicated above and n represents a number from 1 to 4,

are cyclised under the action of protons, by customary methods, to give compounds of the formula (VI)

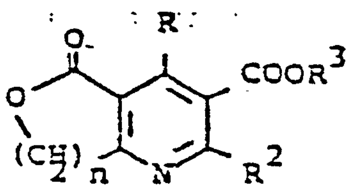

(VI)

in which

R $R^2$, $R^3$ and n have the meaning indicated above.

8) A process for the preparation of medicaments, characterised in that compounds according to Claims 1 and 2 are converted into a suitable form for administration optionally using customary auxiliaries and vehicles.

**Revendications**

1. Esters d'acides pyridine-carboxyliques de formule générale I

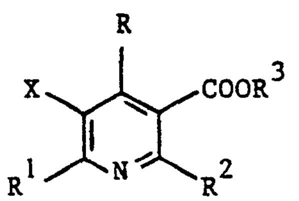

(I)

dans laquelle

R représente un reste phényle ou pyridyle, lereste phényle étant éventuellement mono- ou di-substitué par des groupes nitro, cyano, trifluoreméthyle, le fluor, le chlore, le brome, des groupes alkyle, alcoxy, alkylmercapto contenant chacun 1 à 4 atomes de carbone dans les parties alkyle et alcoxy, ou bien un reste benzoxadiazolyle,

$R^1$ représente l'hydrogène ou un groupe alkyle en $C_1$-$C_4$, un groupe phényle, un groupe benzyle, un groupe acétoxyméthyle, un groupe 2-acétoxyéthyle ou un groupe hydroxyalkyle en $C_1$-$C_4$,

ou bien

$R^1$ forme avec X un cycle de 5 à 7 chaînons contenant en tant que chaînon cyclique un groupe carbonyle et le cas échéant un atome d'oxygène ou d'azote, l'azote portant encore éventuellement un atome d'hydrogène ou un groupe alkyle inférieur en $C_1$-$C_4$,

$R^2$ représente l'hydrogène ou un groupe alkyle droit ou ramifié en $C_1$-$C_4$, phényle, benzyle, acétoxyméthyle, 2-acétoxyéthyle ou hydroxyalkyle en $C_1$-$C_4$,

X représente un groupe nitrile

ou bien

le reste $COR_4$ dans lequel $R^4$ représente un groupe alkyle droit ou ramifié en $C_1$-$C_4$, phényle ou benzyle,

ou bien

le groupe $COOR^5$ dans lequel $R^5$ représente un reste hydrocarboné à chaîne droite, ramifiée ou cyclique, saturé ou insaturé, contenant jusqu'à 10 atomes de carbone, qui est éventuellement interrompu dans la chaîne par un atome d'oxygène et/ou qui est éventuellement substitué par le fluor, le chlore, le brome, des groupes cyano, hydroxy, acétoxy, α-, β- ou γ-pyridyle, phényle, phénoxy, les groupes aryle étant eux-mêmes substitués par le fluor, le chlore, des groupes nitro, trifluorométhyle, alkyle, alcoxy contenant chacun 1 à 4 atomes de carbone ou amino, le groupe amino portant lui-même éventuellement deux substituants identiques ou différents pris parmi les groupes alkyle en $C_1$-$C_4$, phényle ou benzyle, ou bien

$R^5$ peut également représenter l'hydrogène lorsque $R^1$ représente un groupe hydroxyalkyle ou acyloxyalkyle,

ou bien

le groupe $SO_2R^6$ dans lequel $R^6$ représente un groupe alkyle en $C_1$-$C_4$ ou phényle, éventuellement substitué par le fluor, le chlore, un groupe trifluorméthyle, alkyle ou alcoxy contenant chacun 1 à 2 atomes de carbone,

$R^3$ est toujours différent de $R^5$ et représenteunreste hydrocarboné à chaîne droite contenant jusqu'à 6 atomes de carbone, qui est interrompu dans la chaîne par un atome d'oxygène et/ou qui est substitué par le fluor, le chlore, le brome, des groupes cyano, hydroxy, acétoxy, dialkylamino contenant jusqu'à 4 atomes de carbone dans chaque groupe alkyle, α-, β- ou γ-pyridyle, phényle ou phénoxy, les groupes aryle pouvant eux-mêmes être substitués par les groupes trifluorométhyle, le fluor, le chlore, des groupes nitro, alkyle ou alcoxy contenant chacun 1 à 4 atomes de carbone,

ainsi que leurs sels acceptables pour l'usage pharmaceutique.

2. Le 5,7-dihydro-2-méthyl-4-(3-nitrophényl)-5-oxo-furo-[3,4-b]pyridine-3-carboxylate de 2-hydroxyéthyle.

3. Les composés selon les revendications 1 et 2 pour l'utilisation dans le traitement de maladies.

4. Les composés selon les revendications 1 et2 pour l'utilisation dans le traitement de maladies dues à une ischémie et/ou à une hypoxie.

5. Médicament contenant au moins un composé selon les revendications 1 ou 2.

6. Médicament exerçant un effet de protection contre l'hypoxie contenant au moins un composé selon les revendications 1 ou 2.

7. Procédé de préparation d'esters d'acides pyridine-carboxyliques de formule générale I

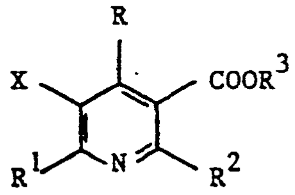

(I)

dans laquelle

R représente un reste phényle ou pyridyle, le reste phényle portant éventuellement un ou deux substituants choisis parmi les groupes nitro, cyano, trifluorométhyle, le fluor, le chlore, le brome, les groupes alkyle, alcoxy, alkylmercapto contenant chacun 1 à 4 atomes de carbone dans les parties alkyle et alcoxy, ou bien un reste benzoxadiazolyle,

$R^1$ représente l'hydrogène ou un groupe alkyle en $C_1$-$C_4$, le groupe phényle, le groupe benzyle, le groupe acétoxyméthyle, le groupe 2-acétoxyéthyle ou un groupe hydroxyalkyle en $C_1$-$C_4$, ou bien

$R^1$ forme avec X un cycle de 5 à 7 chaînons contenant en tant que chaînon cyclique un groupe carbonyle et le cas échéant un atome d'oxygène ou d'azote, l'azote portant encore éventuellement un atome d'hydrogène ou un groupe alkyle inférieur en $C_1$-$C_4$,

$R_2$ représente l'hydrogène ou un groupe alkyle droit ou ramifié en $C_1$-$C_4$, phényle, benzyle, acétoxyméthyle, 2-acétoxyéthyle ou hydroxyalkyle en $C_1$-$C_4$,

X représente un groupe nitrile

ou bien

le reste $COR^4$ dans lequel $R^4$ représente un groupe alkyle droit ou ramifié en $C_1$-$C_4$, phényle ou benzyle,

ou bien

le groupe $COOR^5$ dans lequel $R^5$ représente un reste hydrocarboné à chaîne droite, ramifiée ou cyclique, saturé ou insature, contenant jusqu'à 10 atomes de carbone, qui est éventuellement interrompu dans la chaîne par un atome d'oxygène et/ou qui est éventuellement substitué par le fluor, le chlore, le brome, des groupes cyano, hydroxy, acétoxy, α-, β- ou γ-pyridyle, phényle, phénoxy, les groupes aryle étant eux-mêmes substitués par le fluor, le chlore, des groupes nitro, trifluorométhyle, alkyle, alcoxy contenant chacun 1 à 4 atomes de carbone ou amino, le groupe amino portant lui-même éventuellement deux substituants identiques ou différents pris parmi les groupes alkyle en $C_1$-$C_4$, phényle ou benzyle, ou bien

$R^5$ peut également représenter l'hydrogène lorsque $R^1$ représente un groupe hydroxyalkyle ou acyloxyalkyle,

ou bien

le groupe $SO_2R^6$ dans lequel $R^6$ représente un groupe alkyle en $C_1$-$C_4$ ou phényle, éventuellement substitué par le fluor, le chlore, des groupes trifluorométhyle, alkyle ou alcoxy contenant chacun 1 à 2 atomes de carbone,

$R^3$ est toujours différent de $R^6$ et représente un reste hydrocarboné à chaîne droite contenant jusqu'à 6 atomes de carbone, qui est interrompu dans la chaîne par un atome d'oxygène et/ou qui est substitué par le fluor, le chlore, le brome, des groupes cyano, hydroxy, acétoxy, dialkylamino contenant jusqu'à 4 atomes de carbone par groupe alkyle, α-, β- ou γ-pyridyle, phényle ou phénoxy, les groupes aryle pouvant eux-mêmes être substitués par des groupes trifluorométhyle, le fluor, le chlore, des groupes nitro, alkyle ou alcoxy contenant chacun 1 à 4 atomes de carbone,

et de leurs sels acceptables pour l'usage pharmaceutique, caractérisé en ce que

A) on fait réagir des dérivés de 1,4-dihydropyridines de formule générale (II)

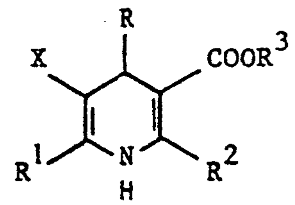

(II)

dans laquelle

R, $R^1$, $R^2$, $R^3$ et X ont les significations indiquées ci-dessus, avec des agents oxydants (déshydrogénants) éventuellement en presence de solvants inertes à des températures de 0 à 200°C, ou bien

B) on estérifie des acides pyridine-carboxyliques de formule générale (III)

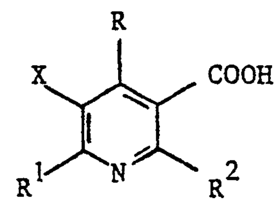

(III)

dans laquelle

R, $R^1$, $R^2$ et X ont les significations indiquées ci-dessus, éventuellement après activation du groupe carboxyle par des modes opératoires décrits dans la littérature scientifique, en esters d'acides carboxyliques de formule générale (I), ou bien

C) dans le cas où $R^1$ et/ou $R^2$ de la formule générale (I) représentent un groupe hydroxyalkyle, on soumet respectivement les mono- et bis-acétoxyalkylpyridines correspondantes aux conditions d'une hydrolyse ménagée et sélective, ou bien

D) dans le cas où X de la formule (I) représente un groupe carboxyle et $R^1$ un groupe hydroxyalkyle,

on saponifie des composés de formule (IV)

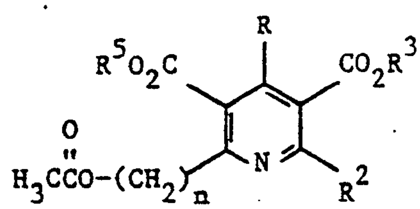

(IV)

dans laquelle

R, $R^2$, $R^3$, $R^5$ et n ont les significations indiquées ci-dessus, en présence d'un alcali, en composés de formule (V), ou bien

E) dans le cas où $R^1$ forme avec X de la formule générale (I) un cycle lactone,

on cyclise des acides hydroxyalkyl-pyridine-carboxyliques de formule générale (V)

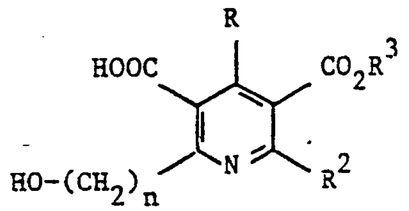

(V)

dans laquelle

R, $R^2$ et $R^3$ ont les significations indiquées ci-dessus et n est un nombre de 1 à 4,

sous l'influence de protons, par des modes opératoires usuels, en composés de formule (VI)

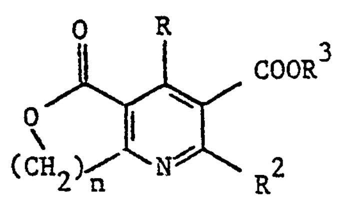

(VI)

dans laquelle

R, $R^2$, $R^3$ et n ont les significations indiquées ci-dessus.

8. Procédé de préparation de médicaments, caractérisé en ce que l'on met des composés selon les revendications 1 et 2, éventuellement par utilisation de produits auxiliaires et véhicules usuels, sous une forme d'administration appropriée.